(19) Europäisches Patentamt
European Patent Office
Office européen des brevets

(11) Veröffentlichungsnummer: **0 063 101**
**B1**

(12) **EUROPÄISCHE PATENTSCHRIFT**

(45) Veröffentlichungstag der Patentschrift: **01.04.87**

(51) Int. Cl.⁴: **C 07 C 147/10**, C 07 C 147/12, C 07 D 307/16, C 07 D 307/38, A 01 N 41/10, A 01 N 53/00

(21) Anmeldenummer: **82810152.7**

(22) Anmeldetag: **06.04.82**

(54) **Nitroaryl-alkylsulfon-derivate als Gametozide.**

(30) Priorität: **14.04.81 CH 2478/81**

(43) Veröffentlichungstag der Anmeldung: **20.10.82 Patentblatt 82/42**

(45) Bekanntmachung des Hinweises auf die Patenterteilung: **01.04.87 Patentblatt 87/14**

(84) Benannte Vertragsstaaten: **AT BE CH DE FR IT LI NL**

(56) Entgegenhaltungen:
**DE - A - 2 049 391**
**DE - A - 2 315 955**
**FR - A - 2 186 965**
**GB - A - 1 128 217**

**CHEMICAL ABSTRACTS, Band 53, Nr. 4, 25. Februar 1959, Spalte 3110b, Columbus, OHio, USA, N.N. VOROZHTSOV, JR. et al.: "Aromatic fluoro derivatives from the chloro derivatives"**
**CHEMICAL ABSTRACTS, Band 55, Nr. 19, 18. September 1961, Spalten 18646h-18647d, Columbus, Ohio, USA V.A.LAVRISHCHEV et al.: "Reaction of alkoxy and aryloxy derivatives of aromatic series with molten urea"**

(73) Patentinhaber: **CIBA-GEIGY AG, Klybeckstrasse 141, CH-4002 Basel (CH)**

(72) Erfinder: **Fankhauser, Ernst, Dr., Route d'Yverdon, CH-1470 Estavayar-le-Lac (CH)**
Erfinder: **Sturm, Elmar, Dr., Klusstrasse 66, CH-4147 Aesch (CH)**

EP 0 063 101 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft die Verwendung von Nitroaryl-alkylsulfon-derivaten der unten definierten Formel I als Wirkstoffe zur Stimulierung des generativen Pflanzenwachstums, insbesondere deren Verwendung zur Erzeugung männlich steriler Pflanzen (Einsatz als Gametozide). Sie betrifft ferner die Verwendung von Nitroaryl-alkylsulfon-derivaten zur Steuerung der Blütenbildung und der damit verbundenen Sekundäreffekte. Darüber hinaus betrifft die Erfindung ein Verfahren zur Gewinnung von Hybrid-Samen.

Als Wirkstoffe sind im Rahmen der vorliegenden Erfindung Verbindungen der Formel I

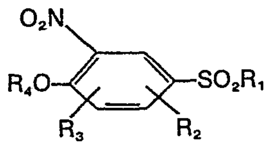

(I)

zu verstehen, worin

$R_1$ $C_1$–$C_6$-Alkyl bedeutet;

$R_2$ und $R_3$ unabhängig voneinander für Wasserstoff, $C_1$–$C_6$-Alkyl, $C_1$–$C_6$-Alkoxy, $C_1$–$C_6$-Haloalkyl, Halogen, Cyano, Nitro, Amino stehen oder orthoständig zueinander als Gruppe $-(CH=CH)_2-$ einen Naphthalinring vervollständigen;

$R_4$ für Wasserstoff, ein Kation oder für die Gruppe $-COR_5$ steht, wobei

$R_5$ $C_1$–$C_{12}$-Alkyl, $C_1$–$C_{12}$-Alkoxy, $C_1$–$C_{12}$-Haloalkoxy, $C_2$–$C_{10}$-Alkenyl, $C_2$–$C_{10}$-Haloalkenyl, $C_2$–$C_6$-Alkinyl, $C_3$–$C_7$-Cycloalkyl, Phenyl, Benzyl, Furyl oder Tetrahydrofuryl bedeutet, und wobei jeder der cyclischen Substituenten unsubstituiert oder ein- oder mehrfach durch gleiche oder verschiedene Substituenten aus der Reihe $C_1$–$C_3$-Alkyl, $C_1$–$C_3$-Alkoxy, $C_1$–$C_3$-Haloalkyl, Halogen, Cyano und Nitro substituiert ist.

Unter den Begriff Alkyl selbst oder als Bestandteil eines anderen Substituenten sind je nach Zahl der angegebenen Kohlenstoffatome beispielsweise folgende Gruppen zu verstehen: Methyl, Ethyl, Propyl, Butyl, Pentyl, Hexyl, Heptyl, Octyl, Nonyl, Decyl, Undecyl oder Dodecyl sowie ihre Isomeren, wie z.B. Isopropyl, Isobutyl, tert.-Butyl, Isopentyl usw. Alkenyl steht z.B. für Vinyl, Propenyl-(1), Allyl, Butenyl-(1), Butenyl-(2), Butenyl-(3) usw., sowie Ketten mit mehreren Doppelbindungen. Alkinyl bedeutet z.B. Propinyl-(1), Propargyl, Butinyl-(1), Butinyl-(2) usw., bevorzugt Propargyl. Haloalkyl steht für einen ein- bis perhalogenierten Alkylsubstituenten, wie z.B. $CHCl_2$, $CH_2Cl$, $CCl_3$, $CCF_3$, $CH_2CH_2Cl$, usw. Unter Halogen soll hier und im folgenden Fluor, Chlor, Brom oder Jod, vorzugsweise Chlor oder Brom verstanden werden. Cycloalkyl steht z.B. für Cyclopropyl, Cyclobutyl, Cyclopentyl, Cyclohexyl oder Cycloheptyl, bevorzugt für Cyclopentyl und Cyclohexyl.

Haloalkenyl und Haloalkoxy stehen jeweils für eine ein- oder mehrfach durch Halogen substituierte Alkenyl- bzw. Alkoxygruppe, wobei als Halogen Chlor und Brom, insbesondere Chlor bevorzugt sind. Niederalkyl bedeutet hier stets $C_1$–$C_8$-Alkyl.

Unter einem Kation ist beispielsweise das anorganische Kation eines Elements der ersten bis vierten Hauptgruppe des Periodensystems zu verstehen. Typische Vertreter sind die Alkalimetalle wie Lithium, Natrium, Kalium oder die Erdalkalimetalle wie Magnesium, Calcium, Barium oder Elemente wie Aluminium, Zinn oder Blei. Darunter ist ferner das Kation eines Elements der ersten bis achten Nebengruppe zu verstehen, wie z.B. von Chrom, Mangan, Eisen, Kobalt, Nickel, Kupfer, Zink, Silber oder Quecksilber. Bevorzugt sind Alkali- und Erdalkalikationen sowie die Kationen der Elemente aus der dritten und vierten Periode des Periodensystems. Je nach Wertigkeit des Metallkations enthalten die Verbindungen der Formel I ein oder mehrere Phenolatreste. Die Bezeichnung Kation steht auch für organische Kationen wie Ammoniumionen oder für Hydraziniumionen.

Als Ammoniumionen kommen z.B. in Frage: $NH_4$, $NH(Alkyl)_3$, $NH_2(Alkyl)_2$ und $NH_3(Alkyl)$ wie $NH(CH_3)_3$, $NH(C_2H_5)_3$, $NH_2(CH_3)_2$, $NH_2(C_3H_7\text{-}n)_2$, $NH_3CH_3$, $NH_3C_4H_9$-n oder quaternäre Ammoniumionen wie Tetraethyl-, Tetrapropyl-, Tetrabutyl-, Tetrapentyl-, Tetrahexyl-, Tetraheptyl-, Tetraoctyl-, Tetranonyl-, Tetradecyl-, Methyltributyl-, Dimethyldibutyl-, Trimethylbutyl-, Methyltrioctyl-, Benzyltrimethyl-, Benzyltriethyl-, Benzyltripropyl-, Benzyltributyl-, Benzyldimethylhexadecyl-, Benzyldiethylhexadecyl-, Diisobutyl-cresoxyethyldimethylbenzyl-, Trimethylphenyl-, Diphenyldimethyl-, Butyltripropyl-, Tributylphenyl- oder Tricaprylmethylammonium.

Als Hydraziniumionen kommen unsubstituierte und substituierte Hydraziniumverbindungen in Frage wie $NH_2NH_3$, $NH_2N(Alkyl)_3$, $NH_2NH(Alkyl)_2$, $NH_2NH_2(Alkyl)$ usw.

Unter den Ammoniumkationen sind neben dem $NH_4$ insbesondere solche des Typs $NH_{(4\text{-}a)}$ $(\text{Niederalkyl})_a^+$ mit a = 1, 2, 3, 4 bevorzugt und unter diesem vor allem Tetraalkylammoniumionen, wie $N(CH_3)_4^+$, $N(C_2H_5)_4^+$, $N(C_4H_9\text{-}n)_4^+$, $N(CH_3)_2(C_2H_5)_2^+$, $N(C_3H_7\text{-}n)_4^+$, $N(C_3H_7\text{-}i)_4^+$, usw.

Die Verbindungen der Formel I sind bei Raumtemperatur stabile Öle, Harze oder überwiegend Feststoffe, die sich durch sehr wertvolle pflanzenwachstumsregulierende Eigenschaften auszeichnen. Sie lassen sich daher bevorzugt auf dem Agrarsektor oder verwandten Gebieten zur gezielten Beeinflussung der Blütenbildung bei Pflanzen einsetzen und eignen sich ferner zur Bekämpfung von phytopathogenen Mikroorganismen.

Eine bevorzugte Gruppe von Wirkstoffen bilden Verbindungen der Formel I, worin $R_1$ für $C_1$–$C_3$-Alkyl steht, $R_2$ und $R_3$ unabhängig voneinander für Wasserstoff, $C_1$–$C_3$-Alkyl, $C_1$–$C_3$-Alkoxy, $C_1$–$C_3$-Haloalkyl, Halogen, Cyano, Nitro, Amino stehen oder orthoständig zueinander als Gruppe $-(CH=CH)_2-$ einen Naphthalinring vervollständigen; $R_4$ für Wasserstoff, ein Alkali- oder Erdalkalikation oder für die Gruppe $-COR_5$ steht, wobei $R_5$ $C_1$–$C_6$-Alkyl, $C_1$–$C_6$-Alkoxy, $C_1$–$C_6$-Haloalkoxy, $C_2$–$C_5$-Alkenyl, $C_2$–$C_5$-Haloalkenyl, $C_2$–$C_5$-Alkinyl, $C_3$–$C_7$-Cycloalkyl, Phenyl, Benzyl, Furyl oder Tetrahydrofuryl bedeutet, und wobei jeder der cyclischen Substituenten unsubstituiert oder durch gleiche oder verschiedene Substituenten aus der Reihe Methyl, Ethyl, Methoxy, Ethoxy, Halomethyl, Fluor, Chlor, Brom, Cyano und Nitro substituiert ist. Innerhalb dieser Gruppe von Wirkstoffen sind insbesondere diejenigen Verbindungen bevorzugt, worin die Gesamtzahl der Kohlenstoffatome in den Substituenten $R_1$, $R_2$, $R_3$ und $R_4$ die Zahl 8 nicht überschreitet.

Bevorzugt werden auch Wirkstoffe der Formel I, worin $R_1$ für Methyl oder Ethyl steht, $R_2$ Wasserstoff bedeutet, $R_3$ für Wasserstoff, Methyl, Methoxy, Trifluormethyl, Brom, Cyano, Nitro oder Amino steht,

$R_4$ Wasserstoff oder die Gruppe $-COR_5$ repräsentiert, wobei $R_5$ $C_1$–$C_3$-Alkyl, $C_1$–$C_3$-Alkoxy, $C_1$–$C_3$-Haloalkoxy, $C_2$–$C_5$-Alkenyl, $C_2$–$C_5$-Haloalkenyl, $C_2$–$C_5$-Alkinyl, $C_3$–$C_7$-Cycloalkyl, Phenyl, Benzyl, Furyl oder Tetrahydrofuryl bedeutet.

Unter der letztgenannten Gruppe von Wirkstoffen sind besonders diejenigen Verbindungen der Formel I bevorzugt, worin $R_1$ für Methyl steht, $R_2$ und $R_3$ Wasserstoff bedeuten und $R_4$ Wasserstoff oder die Gruppe $-COR_5$ repräsentiert, wobei $R_5$ $C_1$–$C_3$-Alkyl, $C_1$–$C_3$-Alkoxy, $C_2$–$C_5$-Alkenyl, $C_2$–$C_5$-Haloalkenyl, $C_3$–$C_7$-Cycloalkyl, Phenyl, Benzyl, Furyl oder Tetrahydrofuryl bedeutet.

Besonders bevorzugt sind Wirkstoffe der Formel I, worin $R_1$ Methyl bedeutet, $R_2$ und $R_3$ unabhängig voneinander für Wasserstoff, Methyl, Ethyl, Methoxy, Brom, Cyano, Nitro, Amino stehen oder orthoständig zueinander als Gruppe $-(CH=CH)_2-$ einen Naphthalinring vervollständigen und $R_4$ für Wasserstoff oder bevorzugt für ein Hydrazinium-, insbesondere Ammoniumion steht.

Als typische Vertreter der Verbindungen der Formel I gelten Substanzen wie
4-Hydroxy-3-nitrophenyl-methylsulfon,
4-[3-chlor-n-propylcarbonyloxy]-3-nitrophenyl-methylsulfon,
4-Hydroxy-3-nitrophenyl-methylsulfon-tetra-(n-butyl)ammoniumsalz,
4-Acetyloxy-3-nitrophenyl-methylsulfon,
4-Cyclopropylcarbonyl-3-nitrophenyl-methylsulfon,
4-Hydroxy-3-nitrophenyl-ethylsulfon,
4-Acryloxy-3-nitrophenyl-methylsulfon,
4-Trichloracryloxy-3-nitrophenyl-methylsulfon,
4-n-Propylcarbonyloxy-3-nitrophenyl-methylsulfon,
4-Ethylcarbonyloxy-3-nitrophenyl-methylsulfon,
4-Phenylcarbonyloxy-3-nitrophenyl-methylsulfon,
4-Methoxymethylcarbonyloxy-3-nitrophenyl-methylsulfon,
4-Cyclohexylcarbonyloxy-3-nitrophenyl-methylsulfon,
4-Chlormethylcarbonyloxy-3-nitrophenyl-methylsulfon
und
4-Methoxycarbonyloxy-3-nitrophenyl-methylsulfon.

Wirkstoffe der Formel I werden dadurch hergestellt, dass man ein Arylhalogenid der Formel II

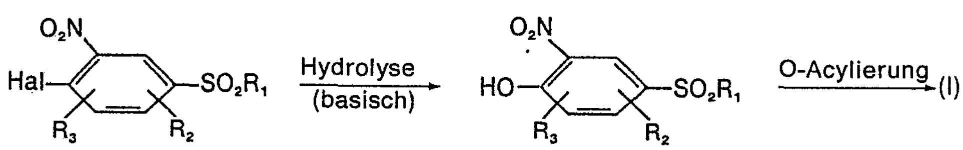


(II) (III)

in Gegenwart einer Base zu einem aromatischen Alkohol der Formel III hydrolysiert und sofern gewünscht mit einem reaktionsfähigen Derivat, vorzugsweise dem Anhydrid oder Säurehalogenid, insbesondere dem Säurechlorid oder Säurebromid der Säure der Formel IV [$R_5COOH$ (IV)] an der phenolischen OH-Gruppe, in an sich bekannter Weise zu O-substituierten Produkten der Formel I acyliert oder durch Neutralisation der sauren OH-Gruppe mit einer Base in ein Salz der Formel I überführt und das gebildete Produkt auf übliche Weise isoliert. In den Formeln II, III und IV haben die Substituenten $R_1$ bis $R_5$ die unter Formel I angegebenen Bedeutungen. Hal steht für Halogen. Geeignete Basen sind beispielsweise wässrige Hydrazinium- bzw. Ammoniumhydroxidderivate.

Bei der Acylierungs-Reaktion können reaktionsinerte Lösungs- oder Verdünnungsmittel eingesetzt werden. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander. In manchen Fällen kann das Acylierungsmittel selbst als Lösungsmittel eingesetzt werden.

Bei der O-Acylierungsreaktion kann die Gegenwart eines Reaktionskatalysators wie beispielsweise Dimethylformamid von Vorteil sein.

Die Reaktionstemperaturen liegen im allgemeinen zwischen 0° und 180 °C, vorzugsweise 0° und 150 °C oder am Siedepunkt des Lösungsmittels oder Lösungsmittelgemisches. In manchen Fällen ist die Verwendung von säurebindenden Mitteln oder Kondensationsmitteln vorteilhaft. Als solche kommen organische und anorganische Basen in Betracht, z.B. tertiäre Amine wie Trialkylamine

(Trimethylamin, Triethylamin, Tripropylamin usw.), Pyridin und Pyridinbasen (4-Dimethylaminopyridin, 4-Pyrrolidinylaminopyridin usw.), Oxide und Hydroxide, Carbonate und Hydrogencarbonate von Alkali- und Erdalkalimetallen sowie Alkaliacetate.

Entstehender Halogenwasserstoff kann in manchen Fällen auch mittels Durchleiten von Inertgas, z.B. Stickstoff, aus dem Reaktionsgemisch vertrieben werden oder durch Zusatz von Molekularsieb aus dem Gemisch entfernt werden.

Biologisch aktive Nitroaryl-alkylsulfon-derivate sind bereits bekannt. So gehören die Verbindungen der Formel I zu einer Gruppe von Nitroaryl-alkylsulfonen, die in der Britischen Patentschrift 1 128 217 als Herbizide zur Bekämpfung von Schadpflanzen offenbart sind. 5-Brom-4-hydroxy-3-nitrophenyl-methylsulfon, 5-Jod-4-hydroxy-3-nitrophenyl-methylsulfon und 5-Chlormethyl-4-hydroxy-3-nitrophenyl-methylsulfon sind in der zitierten Patentschrift namentlich genannt, die übrigen Verbindungen der Formel I nur in allgemeinster Form umfasst. Herstellung sowie chemische und physikalische Eigenschaften von 4-Hydroxy-3-nitrophenyl-methylsulfon sind aus Chem. Abstr. 53, 3110d (1959) und von 4-Acetyloxy-3-nitrophenyl-methylsulfon aus Zhur. Obsh. Khim 30, 3064–3072 (1960) bekannt.

Es wurde nunmehr überraschend gefunden, dass Nitroaryl-alkylsulfon-derivate der Formel I bzw. Mittel, die diese Wirkstoffe enthalten, sich vor allem dadurch auszeichnen, dass sie gezielt in die Physiologie von Pflanzen eingreifen. Ein derartiger Eingriff in die physiologischen Vorgänge der Pflanzenentwicklung macht die Wirkstoffe der Formel I für verschiedene Zwecke verwendbar, z.B. für solche, die mit der Ertragssteigerung bei Nutzpflanzen und insbesondere der Arbeitseinsparung bei der Züchtung und Produktion von Hybridsaatgut im Zusammenhang stehen.

Nach bisherigen Erfahrungen gilt für die Applikation von Wachstumsregulatoren, dass die Aktivsubstanzen eine oder mehrere unterschiedliche Wirkungen bei den Pflanzen hervorrufen können. Diese verschiedenartigen Wirkungen hängen im wesentlichen vom Zeitpunkt der Anwendung, d.h. vom physiologischen Zustand des Samens oder vom Entwicklungszustand der Pflanze, der Art der Applikation sowie insbesondere von den angewendeten Konzentrationen ab. Derartige Effekte sind wiederum je nach Pflanzenart verschieden. Die Applikation von Verbindungen der Formel I eröffnet nunmehr die Möglichkeit das Wachstum von Pflanzen in gewünschter Weise zu beeinflussen.

Mit den Nitroaryl-alkylsulfon-derivaten der Formel I bzw. mit agrochemischen Mitteln, die diese Verbindungen als Wirkstoff enthalten, lässt sich das generative Wachstum einer ganzen Reihe mono- und dicotyler Pflanzen steuern, wobei das vegetative Wachstum in weiten Konzentrationsbereichen für die Kulturen positiv beeinflusst wird. Bei sehr hohen Aufwandmengen können phytotoxische Effekte auftreten. Der Einfluss auf das generative Pflanzenwachstum macht sich, je nach Pflanzenkultur, unterschiedlich bemerkbar.

Eine im Vordergrund stehende Art der Steuerung des generativen Wachstums beruht auf der besonderen Eigenschaft der hierin beschriebenen Nitroaryl-alkyl-sulfon-derivate, bei verschiedenartigen Kulturpflanzen, insbesondere bei der Anwendung in Monocotyledonen, wie Weizen, Roggen, Gerste, Hafer, Reis, Mais, Sorghum-Hirse, Futtergräser usw., aber auch bei anderen Pflanzen, wie bei Sonnenblumen oder Baumwolle eine gametozide Wirkung zu entfalten. Diese führt zu einer männlichen Sterilität der Pflanze, ohne die Fruchtbarkeit der weiblichen Blütenteile merklich zu beeinflussen. Bei zahlreichen Kulturpflanzen ist gleichzeitig eine Zunahme der Blütenansätze und/oder die Ausbildung parthenokarper Früchte (z.B. bei Tomaten) zu beobachten. Die männliche Sterilität zeigt sich entweder in einer aktuellen männlichen Sterilität, nämlich darin, dass die männlichen Blütenteile überhaupt nicht gebildet werden oder darin, dass die Blütenpollen steril sind, oder sie zeigt sich in ,einer funktionellen männlichen Sterilität, bei der die männlichen Blütenteile zwar noch gebildet werden, aber nicht in der Lage sind, eine Befruchtung herbeizuführen. Die Wirkstoffe der Formel I können daher auch Protogynie hervorrufen, d.h. es werden vorzeitig befruchtungsfähige weibliche Blütenteile ausgebildet oder das Heranwachsen der männlichen Blütenteile zeitlich so verzögert, dass eine Fremdbestäubung mit ausgewählten Blütenpollen durchgeführt werden kann.

Diese gametoziden Effekte lassen sich besonders vorteilhaft bei der Züchtung und Feldproduktion von Hybrid-Saatgut einsetzen. Hybrid-Saatgut spielt bei den Hauptnahrungsnutzpflanzen und Zierpflanzen eine bedeutende Rolle. Hybride sind üblicherweise vitaler als reine Arten und liefern höhere Erträge, als die ertragsreichste Elternsorte.

Bei der Hybridisierung kreuzt der Züchter zwei oder mehr sorgfältig ausgewählte Inzuchtlinien in einem experimentell ermittelten Schema und erhält auf diese Weise Hybridsaatgut dessen Pflanzen gesteigerte Wüchsigkeit und Leistung aufweisen.

Bei den einhäusigen synözischen Maispflanzen lässt sich eine Hybridisierung auch auf konventionelle Weise durchführen, da männliche und weibliche Blütenteile an unterschiedlichen Stellen der Pflanze ausgebildet werden (Dikline Blüten). Die Antheren, die Pollen liefern, bilden die Spitze der Maispflanze, während die kolbenförmige, weibliche Infloreszenz mit den Narbenfäden unterhalb der Pflanzenmitte ausgebildet wird. Um nun $F_1$-Hybride zu züchten werden üblicherweise alternierende Reihen von Maispflanzen der Sorten oder homozygoten Linien AA und BB angepflanzt. Zur Sicherstellung, dass der AA-Mais keine Pollen bildet, werden die AA-Pflanzen rechtzeitig, vor der vollständigen Entwicklung der männlichen Blütenstände manuell oder maschinell entfahnt und anschliessend mit Pollen einer BB-Mais-Sorte bestäubt, wobei auf den AA-Pflanzen Saat-

gut eines AB-Hybrids ($F_1$) entsteht. Der hierzu erforderliche Arbeitsvorgang ist nicht nur zeitraubend und aufwendig, sondern führt zwangsläufig zu einer Verletzung der Pflanze und besonders bei maschineller Entmännlichung zu einer unerwünschten Ertragsdepression bei der als weiblicher Elter (Samenträger) dienenden Linie/Sorte.

Bei einhäusigen, synözischen Pflanzen wie Mais lässt sich die Hybridisierung nach der geschilderten, herkömmlichen Methode noch einigermassen wirtschaftlich bewerkstelligen. Wesentlich schwieriger gestaltet sich dieser Vorgang jedoch bei kleinkörnigen Halmfrüchten, insbesondere bei jenen, die Zwitterblüten aufweisen und sich normalerweise selbst- (sog. Selbstbefruchtung) aber auch kreuzbestäuben (sog. Fremdbefruchtung). In diesen Fällen erweist sich das herkömmliche Verfahren als äusserst zeitraubend, arbeitsintensiv, unwirtschaftlich, und es bedarf vor allem speziell ausgebildeter Arbeitskräfte. Kleinkörnige Halmfrucht-Hybride lassen sich nur züchten, wenn die Selbst- und Kreuzbestäubung in der Mutterlinie vollständig unterdrückt wird. In der Praxis muss man dazu jede der winzigen Blüten frühzeitig von Hand öffnen und alle Staubbeutel sorgfältig entfernen und anschliessend die Blüten gegen unerwünschte Fremdbestäubung schützen.

Bei einigen Getreidesorten wie Heizen, Gerste, Hirse sowie Mais und bei Dicotylen wird noch eine weitere Hybridisierungsmethode praktiziert. Bei diesem Verfahren geht man von cytoplasmatisch, männlich sterilen Pflanzen aus und führt Fremdbestäubung durch. Diese cytoplasmatisch sterilen Pflanzen beschränken sich auf Mutterlinien, die dasselbe Cytoplasma aufweisen. Dadurch werden cytoplasmatisch vererbte Schwächen oder Fehler, z. B. mangelnde Resistenz gegen einen bestimmten Krankheitserreger oder Frostempfindlichkeit und ähnliches, bei dieser Methode zwangsläufig auf alle, von dieser Mutterlinie abstammenden Hybride übertragen. Ausserdem erfordert die Hybridisierung mit cytoplasmatisch, männlich sterilen Linien, insbesondere bei kleinkörnigen Halmfrüchten, aber auch in dicotylen Kulturen, aufwendige Massnahmen von erhöhtem Schwierigkeitsgrad.

Unabhängig von der Methode, setzt die Züchtung von Hybrid-Saatgut stets die Erzeugung männlich steriler und weiblich intakter Pflanzen voraus. Eine einfache, praktikable und wirtschaftliche Lösung zur Herstellung einer selektiven männlichen Sterilität bietet der Einsatz von chemischen Sterilisierungsmitteln (Gametoziden). Nitroaryl-alkylsulfon-derivate der Formel I zeigen sehr gute männlich-gametozide Eigenschaften und sind daher für diese und verwandte Zwecke geeignet. Ihr Einsatz in Kulturen von Nutzpflanzen lässt die meisten Probleme, die an herkömmliche Hybridisierungsmethoden gekoppelt sind, gar nicht erst in Erscheinung treten.

Zur Erzeugung von männlich sterilen Pflanzen und damit zur Gewinnung von Hybrid-Saatgut kann man im einzelnen folgendermassen vorgehen: Beide Elternpflanzen, die miteinander gekreuzt werden sollen, werden in z. B. alternierenden Reihen gepflanzt. Die als Samenträger oder Mutterpflanze ausgewählte Linie wird zu Beginn der Blütenbildung, aber noch vor Ausbildung der männlichen Blütenteile, mit einem der Wirkstoffe der Formel I behandelt. Auf diese Weise erhält man eine Reihe männlich steriler, aber fruchtbarer weiblicher Elternpflanzen. Die andere Reihe bleibt unbehandelt und dient als Pollenspender. Ihre männlichen Blütenteile gelangen zur vollen Ausbildung und liefern die Pollen zur Bestäubung der Mutterpflanze bzw. des Samenträgers. Die von der Mutterpflanze erzeugten Samenkörner stellen das Hybridsaatgut dar und können auf konventionelle Weise geerntet werden. Die Körner der männlichen Elternpflanzen werden separat geerntet und anderweitig genutzt.

Das beschriebene Verfahren zur Erzeugung von männlich sterilen Pflanzen bzw. zur Gewinnung von Hybridsaatgut ist Bestandteil der vorliegenden Erfindung.

Darüber hinaus bewirken die Wirkstoffe der Formel I noch weitere wachstumsregulierende Effekte, beispielsweise eine zeitliche Steuerung der Blütenbildung und in der Folge eine kontrollierte Samen- oder Fruchtreifung. Diese Art der Blütenstimulation ist insbesondere bei solchen Pflanzensorten von wirtschaftlichem Interesse, die gleichzeitig blühen und Früchte tragen. So könnte beispielsweise die Behandlung von Avocado- oder Baumwollpflanzen mit Verbindungen der Formel I einerseits zu einer vorteilhaften Zunahme der Zahl der Blütenstände führen, andererseits der Blüh- und Reifprozess einem kontrollierten Rhythmus unterworfen werden. Auf diese Weise könnte nicht nur eine Ertragssteigerung erreicht werden, sondern eine rationellere Gestaltung der Ernte und somit eine günstigere Vermarktung.

Diese Art der Stimulation der Blütenbildung spielt auch bei der Obsterzeugung eine entscheidende Rolle. Im allgemeinen beobachtet man nämlich beim Obstanbau, genetisch oder durch Aussenfaktoren bedingt, meist einen jährlichen Wechsel zwischen Ertrags- und Ausfalljahren. Diese Alternanz ist die Folge eines Missverhältnisses zwischen dem Triebwachstum und der Blütenanlage, indem die Ernährung des Fruchtbehangs zuviele Assimilate auf Kosten der Bildung von Blütenknospen beansprucht. Die konventionellen Massnahmen zur Verbesserung der Obstqualität bestehen in einer zeitraubenden mechanischen Ausdünnung von ganz jungen Früchten sowie durch wachstumsregulierenden Schnitt der sogenannten wilden Triebe; dies führt jedoch zwangsläufig zu einer Verletzung der Bäume und zu geringen Erträgen. Durch den Einsatz von Nitroaryl-alkylsulfon-derivaten der Formel I lässt sich das Triebwachstum weitgehend zugunsten des generativen Wachstums unterdrücken, so dass die Wuchskraft vermehrt zur Blüten- und Fruchtbildung beiträgt. Auf diese Weise kann nicht nur die Obstqualität, sondern auch der Ertrag verbessert und eine Phasenverschiebung in der Alternanz erzeugt werden.

In manchen Fällen bewirkt die Applikation von Verbindungen der Formel I eine deutliche Verlängerung der Blühperiode, dadurch kann die Möglichkeit der Bestäubung aller Blüten erhöht werden. Eine zeitliche Dehnung der Blühphase ist auch bei einer ganzen Anzahl von Zierpflanzen, insbesondere bei Blumen wünschenswert.

Bei einer ganzen Reihe unterschiedlicher Kulturpflanzen beobachtet man nach deren Behandlung mit Wirkstoffen der Formel I parallel zur männlichen Sterilisierung einen positiven Einfluss auf die weiblichen Blütenstände. Auf diese Weise wird oftmals die Zahl der weiblichen Blüten pro Blütenstand oder pro Pflanze und somit auch der Ertrag erhöht. Derartige Effekte sind auch bei kleinkörnigen Halmfrüchten (wie Gerste), Gurkengewächsen, Sonnenblumen, Hülsenfrüchten (wie Soja), baumartigen Gewächsen und Zierpflanzen (wie Compositen) zu verzeichnen. In einigen Fällen treten weitere, verwandte wachstumsregulierende Wirkungen auf. Daneben kann bei gewissen Pflanzensorten auch eine verringerte Frostempfindlichkeit und eine erhöhte Resistenz gegenüber Krankheitserregern festgestellt werden.

Die vorliegende Erfindung betrifft somit auch die Verwendung von Nitroaryl-alkylsulfon-derivaten der Formel I oder von Mitteln, die diese Verbindungen als Aktivsubstanzen enthalten, zur Steuerung des Pflanzenwuchses, insbesondere zur Erzeugung einer Sterilität der männlichen Blütenteile (Einsatz als Gametozide) und/oder zur Förderung der weiblichen Blütenteile und aller daraus resultierender Sekundäreffekte wie z.B. Ertragssteigerung, Verlängerung der Blühdauer, vermehrte Blütenbildung, Steuerung der Fruchtbildung oder des Reifeprozesses und ähnliches.

Darüber hinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet. Bestandteil dieser Erfindung sind auch alle neuen Wirkstoffe im Umfang der Formel I, einschliesslich ihres hierin beschriebenen Herstellungsverfahrens.

Es wurde ferner festgestellt, dass Verbindungen der Formel I ein für praktische Bedürfnisse sehr günstiges Mikrobizid-Spektrum gegen phytopathogene Pilze und Bakterien aufweisen. Sie besitzen sehr vorteilhafte kurative, präventive und systemische Eigenschaften und lassen sich zum Schutz von Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben. Die Verbindungen der Formel I sind insbesondere gegen Pilzkrankheiten wie Rost, Schorf, echter Mehltau und gegen Fungi imperfecti (z.B. Puccinia, Rhizoctonia, Venturia, Erysiphe, Cercospora) sowie gegen Bakterien aus der Familie der Pseudomonadaceae, insbesondere Xanthomonas-Arten wirksam. Sie können ferner als Beizmittel zur Behandlung von Saatgut (Früchte, Knollen, Körner) und Pflanzenstecklingen zum Schutz vor Bakterien- und Pilzinfektionen sowie gegen andere im Erdboden auftretende phytopathogene Mikroorganismen eingesetzt werden.

Als Zielkulturen für die hierin offenbarten Indikationsgebiete gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Äpfel, Birnen, Pflaumen, Pfirsiche, Mandeln, Kirschen, Erd-, Him- und Brombeeren); Hülsenfrüchte: (Bohnen, Linsen, Erbsen, Soja); Ölkulturen: Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Lein, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen).

Nitroaryl-alkylsulfon-derivate können pre- oder postemergent appliziert werden. Bevorzugt ist jedoch ein post-emergenter Einsatz, d.h. nach dem Auflaufen der Pflanzen.

Die Aufwandmengen richten sich nach dem gewünschten wachstumsregulierenden Effekt und sollten vorteilhafterweise, je nach Entwicklungsstadium und Pflanzengattung, d.h. je nach Applikationszeitpunkt, experimentell bestimmt werden.

Bei den verschiedenen Indikationsgebieten gelten folgende Faustregeln:

Zur Wuchsregulierung, insbesondere zur Erzielung einer gametoziden Wirkung, liegen die Aufwandmengen im allgemeinen pro Applikation zwischen 0,05 und 12, vorzugsweise 0,5 bis 8, insbesondere 1 bis 4 kg Aktivsubstanz pro ha. Als günstiger Applikationszeitpunkt, insbesondere bei Getreidekulturen, gilt die Periode nach dem Auflaufen, aber noch bevor sich Ähren und Antheren zeigen bzw. das 5½ Blattstadium oder die beginnende Blütenbildung.

Soll die Zahl der Blütenstände vermehrt oder die Blühphase verlängert werden, z.B. bei Sonnenblumen, Baumwolle, Gurkengewächsen (wie Kürbis, Gurken, Melonen), Hülsenfrüchten (wie Bohnen, Linsen, Erbsen, Soja) oder Zierpflanzen, so liegen vorteilhafte Dosierungen bei 0,5 bis 4 kg AS/ha, wobei die Applikation notwendigerweise vor dem Blühbeginn, insbesondere vor Beginn der Knospenbildung erfolgt.

Bei Pflanzen wie z.B. der Baumwolle, bei der Blüten und Früchte gleichzeitig auftreten ist Splitapplikation angezeigt, d.h. die Applikation wird bevorzugt mit kleineren Aufwandmengen periodisch wiederholt. Im allgemeinen hängen die

Aufwandmengen auch von der Applikationsart ab und liegen bei der einfachen Blattapplikation bevorzugt zwischen 2 bis 6 kg AS/ha, bei der Splitapplikation zwischen 2 bis 4 kg AS/ha und bei der Boden-(Drench)-applikation bei 3 bis 12 kg AS/ha, je nach Bodentyp.

In der Saatbeizung liegen die Aufwandmengen bei etwa 0,02 bis 1 kg AS pro 100 kg Saatgut. Werden die Verbindungen der Formel I als Mikrobizide eingesetzt so liegen vorteilhafte Aufwandmengen bei 0,5 bis 5 kg pro Hektar. Die Anwendungskonzentration richtet sich in der Praxis jedoch nach dem Befallsdruck für den zu bekämpfenden Mikroorganismus.

Die beschriebenen Aufwandmengen und Applikationsarten sind ein Teil der vorliegenden Erfindung.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Wird dieses Verfahren rechtzeitig zu Beginn der Blüte durchgeführt, so kann auf diese Weise eine sehr selektive männliche Sterilität erzeugt werden. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden). In den Fällen, in denen ein Salz der Formel I formuliert wird, kann auf dessen Isolierung verzichtet werden, da auch die wässrige Lösung eines Phenols der Formel I, das zuvor durch eine Base neutralisiert worden war, direkt mit weiteren Formulierungshilfsstoffen gemischt werden kann.

Als Lösungsmittel können in Frage kommen:

Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; oder Wasser. Der Zusatz von mineralischen Ölen oder von pflanzlichen Ölen ist besonders bei Formulierungen vorteilhaft, die bei der Blattapplikation eingesetzt werden.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$–$C_{22}$), wie z.B. die Na- oder K-Salze der Öl- oder Stearinsäure, oder von natürlichen Fettsäuregemischen, die z.B. aus Kokosnuss- oder Tallöl gewonnen werden können. Ferner sind auch die Fettsäure-methyltaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8–22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4–14)-Ethylenoxid-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykolethergruppen und 10 bis 100 Propylenglykolethergruppen enthaltenden Polyethylenoxid-Addukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykol-Einheiten.

Als Beispiele nichtionischer Tenside seien Nonylphenolpolyäthoxyethanole, Ricinusölpolyglycolether, Polypropylen-Polyethylenoxid-Addukte, Tributylphenoxypolyethoxyethanol, Polyethylenglykol und Octylphenoxypolyethoxyethanol erwähnt.

Ferner kommen auch Fettsäureester von Polyoxyäthylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:

«Mc Cutcheon's Detergents and Emulsifiers Annual» MC Publishing Corp., Ridgewood New Jersey, 1980

Sisely and Wood, «Encyclopedia of Surface Active Agents», Chemical Publishing Co., Inc. New York, 1980.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% eines festen oder flüssigen Zusatzstoffes. Vorteilhafterweise sind 0 bis 25%, vorzugsweise 0,1 bis 25% eines Tensides unter den Zuschlagstoffen enthalten.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht. RT bedeutet Raumtemperatur, h steht für Stunde, Fp. für Schmelzpunkt.

Herstellungsvorschriften:

a) Herstellung von

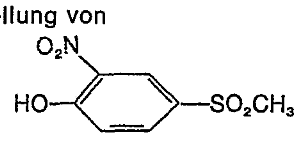

(2.1)

4-Hydroxy-3-nitrophenyl-methylsulfon

In 4 kg 30%iger Natronlauge werden unter Rühren 25,6 kg 4-Chlor-3-nitro-phenyl-methylsulfon langsam eingetragen und die Mischung laufend mit Wasser verdünnt, bis ein Volumen von ca. 50 Liter erreicht ist. Die Reaktionsmischung wird auf 90° erhitzt und innerhalb von 3 bis 4 h werden weitere 29 kg 30%ige Natronlauge unter Rühren zugesetzt. Anschliessend erhitzt man 90 Minuten auf 103°. Nach dem Abkühlen auf ca. 60° wird die Lösung mit 16%iger Schwefelsäure auf einen pH-Wert von ca. 1,5 eingestellt. Dabei fällt das Produkt aus. Es wird abfiltriert, gewaschen und aus Ethanol umkristallisiert. Ausbeute: 24 kg farbloser Kristalle. Fp. 166–168°.

b) Herstellung von

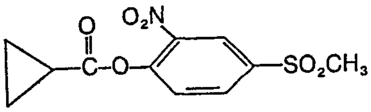

(1.14)

4-Cyclopropylcarbonyloxy-3-nitrophenyl-methylsulfon

32,6 g 4-Hydroxy-3-nitrophenyl-methylsulfon werden in 250 ml Tetrahydrofuran suspendiert und 21 ml Triethylamin zugesetzt. Zu der entstandenen gelben Lösung lässt man 15,7 g Cyclopropancarbonsäurechlorid unter Rühren zutropfen. Nach Abklingen der schwach exothermen Reaktion rührt man noch 3 h bei RT weiter, anschliessend wird gebildetes Triethylammoniumchlorid abfiltriert und das Filtrat eingedampft. Man erhält 42 g eines zähen Öls, das nach Digerieren mit Petrolether 40,5 g des hellgelben, kristallinen Produkts ergibt. Fp. 114–116°.

c) Herstellung von

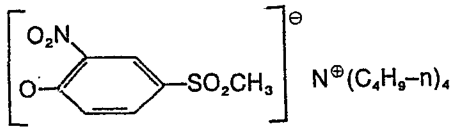

(2.20)

4-Hydroxy-3-nitrophenyl-methylsulfon-tetra(n-butyl)ammoniumsalz

Eine Lösung von 13,5 g 4-Hydroxy-3-nitrophenyl-methylsulfon in 100 ml Ethanol wird mit 41 g einer 40%igen, wässrigen Tetrabutylammoniumhydroxid-Lösung versetzt und die entstehende, tiefgelbe Lösung am Rotationsverdampfer bis zur Trockene eingedampft. Man erhält 28 g einer gelben Kristallmasse. Fp. 98–100 °C.

Auf analoge Weise lassen sich auch die Verbindungen aus den nachfolgenden Tabellen 1 und 2 herstellen.

Tabelle 1: Verbindungen der Formel V

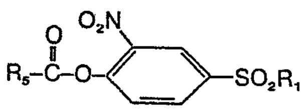

(V)

| Verb. Nr. | $R_1$ | $R_5$ | physikal. Konst. |
|---|---|---|---|
| 1.1 | $C_2H_5$ | $CCl=CCl_2$ | Fp. 89–91° |
| 1.2 | $CH_3$ | $CCl=CCl_2$ | Fp. 129–131° |
| 1.3 | $C_3H_7(n)$ | $CCl=CCl_2$ | Harz |
| 1.4 | $C_3H_7(i)$ | $CCl=CCl_2$ | Harz |
| 1.5 | $CH_3$ | $CH_3$ | Fp. 139–141° |
| 1.6 | $C_3H_7(n)$ | $CH_3$ | |
| 1.7 | $C_3H_7(i)$ | $CH_3$ | |
| 1.8 | $C_2H_5$ | $CH_3$ | Fp. 87–90,5° |
| 1.9 | $CH_3$ | $C_2H_5$ | Fp. 88–90° |
| 1.10 | $CH_3$ | $C_3H_7(n)$ | Fp. 83–85° |
| 1.11 | $CH_3$ | $C_3H_7(i)$ | Fp. 77–79° |
| 1.12 | $C_2H_5$ | $C_2H_5$ | Fp. 79–82° |
| 1.13 | $C_4H_9(n)$ | $CH_3$ | |
| 1.14 | $CH_3$ | Cyclopropyl | Fp. 114–116° |
| 1.15 | $CH_3$ | Cyclobutyl | |
| 1.16 | $CH_3$ | Cyclopentyl | |
| 1.17 | $CH_3$ | Cyclohexyl | Fp. 110–112° |
| 1.18 | $CH_3$ | Cycloheptyl | |
| 1.19 | $CH_3$ | $C_4H_9(n)$ | Fp. 63–65° |
| 1.20 | $CH_3$ | $CH_2C(CH_3)_2CH_3$ | Fp. 89–91° |
| 1.21 | $C_2H_5$ | $C_4H_9(n)$ | |
| 1.22 | $C_2H_5$ | $CH_2C(CH_3)_2CH_3$ | |
| 1.23 | $CH_3$ | $(CH_2)_{10}CH_3$ | Fp. 84–87° |
| 1.24 | $CH_3$ | $CH=CH_2$ | Fp. 114–117° |
| 1.25 | $C_2H_5$ | $CH=CH_2$ | |
| 1.26 | $C_3H_7(n)$ | $CH=CH_2$ | |
| 1.27 | $C_2H_5$ | $CH=CHCH_3$ | Fp. 71–75° |
| 1.28 | $CH_3$ | $CH=CHCH_3$ | Fp. 100–102° |
| 1.29 | $C_2H_5$ | $CH=C(CH_3)_2$ | |
| 1.30 | $C_2H_5$ | $C(CH_3)=CHCH_3$ | |
| 1.31 | $CH_3$ | $CH=C(CH_3)_2$ | Fp. 88–90° |
| 1.32 | $CH_3$ | $C(CH_3)=CHCH_3$ | Fp. 87–89° |
| 1.33 | $CH_3$ | $CH_2Cl$ | Fp. 108–110° |

Tabelle 1: (Fortsetzung)

| Verb. Nr. | $R_1$ | $R_5$ | physikal. Konst. |
|---|---|---|---|
| 1.34 | $CH_3$ | $C_2H_4Cl$ | Fp. 123–125° |
| 1.35 | $C_2H_5$ | $CH_2Cl$ | Fp. 69–72° |
| 1.36 | $C_2H_5$ | $C_2H_4Cl$ | |
| 1.37 | $CH_3$ | $CHCl_2$ | |
| 1.38 | $CH_3$ | $CCl_3$ | |
| 1.39 | $CH_3$ | $(CH{=}CH)_2CH_3$ | Fp. 112–114° |
| 1.40 | $C_2H_5$ | $(CH{=}CH)_2CH_3$ | Fp. 88–93° |
| 1.41 | $CH_3$ | $(CH_2)_8CH{=}CH_2$ | Fp. 56–58° |
| 1.42 | $C_3H_7$(i) | $CCl{=}CHCH_3$ | |
| 1.43 | $CH_3$ | $CCl\text{-}CHCH_3$ | Fp. 120–122° |
| 1.44 | $CH_3$ | $OCH_3$ | Fp. 163–165° |
| 1.45 | $C_2H_5$ | $OCH_3$ | |
| 1.46 | $C_3H_7$(n) | $OCH_3$ | |
| 1.47 | $CH_3$ | $OC_2H_5$ | |
| 1.48 | $CH_3$ | $OC_3H_7$(i) | |
| 1.49 | $CH_3$ | $CH_2Br$ | Fp. 98–100° |
| 1.50 | $CH_3$ | $CH_2J$ | |
| 1.51 | $CH_3$ | $CF_3$ | |
| 1.52 | $CH_3$ | $O(CH_2)_3Cl$ | Fp. 87–89° |
| 1.53 | $CH_3$ | $OC_2H_4Cl$ | |
| 1.54 | $CH_3$ | $OC_4H_2Br$ | |
| 1.55 | $CH_3$ | $CCl{=}CHCl$ | Fp. 129–130° |
| 1.56 | $C_2H_5$ | $CCl{=}CHCl$ | |
| 1.57 | $CH_3$ | $CH_2OC_2H_5$ | |
| 1.58 | $CH_3$ | $(CH_2)_2OCH_3$ | |
| 1.59 | $CH_3$ | $CH_2OCH_3$ | Fp. 91–92° |
| 1.60 | $C_2H_5$ | $CH_2OCH_3$ | |
| 1.61 | $C_2H_5$ | $C_6H_5$ | |
| 1.62 | $CH_3$ | $C_6H_5$ | Fp. 142–144° |
| 1.63 | $CH_3$ | $C_6H_4$(4–Cl) | |
| 1.64 | $CH_3$ | $C_6H_4$(3–Cl) | |
| 1.65 | $CH_3$ | $C_6H_4$(2–Cl) | |
| 1.66 | $C_2H_5$ | $C_6H_4$(4–Cl) | |
| 1.67 | $CH_3$ | $C_6H_4(4\text{–}NO_2)$ | Fp. 205–208° |
| 1.68 | $CH_3$ | $C_6H_3Cl_2$(2,4) | |
| 1.69 | $CH_3$ | $C_6H_4$(4–F) | Fp. 157–159° |
| 1.70 | $CH_3$ | $C_6H_4(4\text{–}CF_3)$ | |
| 1.71 | $C_2H_5$ | $C_6H_4(4\text{–}NO_2)$ | |
| 1.72 | $CH_3$ | $C_6H_3(NO_2)_2$(2,4) | |
| 1.73 | $CH_3$ | $CH{=}CHC_6H_5$ | Fp. 152–155° |
| 1.74 | $C_2H_5$ | 2-Tetrahydrofuryl | Harz |
| 1.75 | $CH_3$ | 2-Tetrahydrofuryl | Harz |
| 1.76 | $C_2H_5$ | 2-Furyl | |
| 1.77 | $CH_3$ | 2-Furyl | Fp. 173–175° |
| 1.78 | $CH_3$ | $(CH_2)_3Cl$ | Fp. 83–85° |
| 1.79 | $CH_3$ | $CHBrCH_3$ | Fp. 89–92° |
| 1.80 | $CH_3$ | $C(CH_3)_3$ | Fp. 117–119° |

Tabelle 2: Verbindungen der Formel I

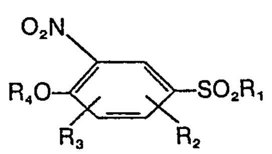

(I)

| Verb. Nr. | $R_1$ | $R_2$ | $R_3$ | $R_4$ | physik. Konst. |
|---|---|---|---|---|---|
| 2.1 | $CH_3$ | H | H | H | Fp. 166–168° |
| 2.2 | $C_2H_5$ | H | H | H | Fp. 138° |
| 2.3 | $CH_3$ | 6–Br | H | H | |

Tabelle 2: (Fortsetzung)

| Verb. Nr. | $R_1$ | $R_2$ | $R_3$ | $R_4$ | physik. Konst. |
|---|---|---|---|---|---|
| 2.4 | $CH_3$ | 6–J | H | H | |
| 2.5 | $CH_3$ | 6–$CH_3$ | H | H | |
| 2.6 | $CH_3$ | 6–Br | 5–Br | H | |
| 2.7 | $CH_3$ | 6–$NO_2$ | H | H | |
| 2.8 | $CH_3$ | 6–$C_3H_7$–i | H | H | |
| 2.9 | $CH_3$ | 6–$CF_3$ | H | H | |
| 2.10 | $C_3H_7$–n | H | H | H | |
| 2.11 | $C_3H_7$–i | H | H | H | |
| 2.12 | $CH_3$ | H | H | 1/2 $Cu^{++}$ | |
| 2.13 | $CH_3$ | H | H | $Na^+$ | |
| 2.14 | $CH_3$ | H | H | 1/3 $Al^{+++}$ | |
| 2.15 | $CH_3$ | H | H | $Mn^{++}$ | |
| 2.16 | $CH_3$ | 6–$CH_3$ | 5–$CH_3$ | H | |
| 2.17 | $CH_3$ | 6–$NH_2$ | H | H | |
| 2.18 | $CH_3$ | 6–Cl | 5–Cl | H | |
| 2.19 | $CH_3$ | H | H | $N(CH_3)_4{}^+$ | Fp. 151–153° |
| 2.20 | $CH_3$ | H | H | $N(C_4H_9\text{–n})_4{}^+$ | Fp. 98–100° |
| 2.21 | $CH_3$ | H | H | $N(C_2H_5)_4{}^+$ | Fp. 130–137° |
| 2.22 | $CH_3$ | H | H | $N(CH_3)_3CH_2C_6H_5{}^+$ | orangegelbes Harz |
| 2.23 | $CH_3$ | H | H | $N(CH_3)_3C_{12}H_{25}{}^+$ | oranges Harz |
| 2.24 | $CH_3$ | H | H | $N(CH_3)_2N(CH_3)_3{}^+$ | oranges Harz |
| 2.24 | $CH_3$ | H | H | $NH_2NH_3$ | |

Formulierungsbeispiele für flüssige Wirkstoffe der Formel I (% = Gewichtsprozent)

| 1. Emulsionskonzentrate | a) | b) | c) |
|---|---|---|---|
| Wirkstoff aus Tabelle 1 oder 2 | 25% | 40% | 50% |
| Ca–Dodecylbenzolsulfonat | 5% | 8% | 6% |
| Ricinusöl-polyäthylenglykol-äther (36 Mol AeO) | 5% | – | – |
| Tributylphenoyl-polyäthylen-glykoläther (30 Mol AeO) | – | 12% | 4% |
| Cyclohexanon | – | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff aus Tabelle 1 oder 2 | 80% | 10% | 5% | 95% |
| Äthylenglykol-mono-methyl-äther | 20% | – | – | – |
| Polyäthylenglykol M G 400 | – | 70% | – | – |
| N-Methyl-2-pyrrolidon | – | 20% | – | – |
| Epoxidiertes Kokos-nussöl | – | – | 1% | 5% |
| Benzin (Siedegrenzen 160–190°C) | – | – | 94% | – |

Die Lösungen sind zur Anwendung in Form kleinster Tropfen geeignet.

| 3. Granulate | a) | b) |
|---|---|---|
| Wirkstoff aus Tabelle 1 oder 2 | 5% | 10% |
| Kaolin | 94% | – |
| Hochdisperse Kieselsäure | 1% | – |
| Attapulgit | – | 90% |

Der Wirkstoff wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff aus Tabelle 1 oder 2 | 2% | 5% |
| Hochdisperse Kieselsäure | 1% | 5% |
| Talkum | 97% | – |
| Kaolin | – | 90% |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

| 5. Spritzpulver | a) | b) | c) |
|---|---|---|---|
| Wirkstoff aus Tabelle 1 oder 2 | 25% | 50% | 75% |
| Na-Ligninsulfonat | 5% | 5% | – |
| Na-Laurylsulfat | 3% | – | 5% |
| Na-Diisobutylnaphthalin-sulfonat | – | 6% | 10% |
| Octylphenolpolyethylenglykol-ether (7–8 Mol AeO) | – | 2% | – |
| Hochdisperse Kieselsäure | 5% | 10% | 10% |
| Kaolin | 62% | 27% | – |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 6. Emulsions-Konzentrat | |
|---|---|
| Wirkstoff aus Tabelle 1 oder 2 | 10% |
| Octylphenolpolyethylenglykolether (4–5 Mol AeO) | 3% |
| Ca-Dodecylbenzolsulfonat | 3% |

| | |
|---|---|
| Ricinusölpolyglykolether (35 Mol AeO) | 4% |
| Cyclohexanon | 20% |
| Xylolgemisch | 50% |
| Kokosnussöl | 10% |

Aus diesem Konzentrat können durch Verdünnen mit Wasser Emulsionen jeder gewünschten Konzentration hergestellt werden.

| 7. Stäubemittel | a) | b) | c) |
|---|---|---|---|
| Wirkstoff aus Tabelle 1 oder 2 | 5% | 8% | 6% |
| Talkum | 95% | – | 44% |
| Kaolin | – | 92% | 50% |

Man erhält anwendungsfertige Stäubemittel, indem der Wirkstoff mit dem Träger vermischt und auf einer geeigneten Mühle vermahlen wird.

| 8. Extruder Granulat | |
|---|---|
| Wirkstoff aus Tabelle 1 oder 2 | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

| 9. Umhüllungs-Granulat | a) | b) |
|---|---|---|
| Wirkstoff aus Tabelle 1 oder 2 | 3% | 5% |
| Polyethylenglykol (M G 200) | 3% | 3% |
| Kaolin | 94% | 92% |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 10. Suspensions-Konzentrat | |
|---|---|
| Wirkstoff aus Tabelle 1 oder 2 | 40% |
| Ethylenglykol | 10% |
| Nonylphenolpolyethylenglykolether (15 Mol AeO) | 6% |
| N-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der feingemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können. Formulierungszusätze, die die Haftung des Wirkstoffs auf der Pflanze erhöhen, wie z.B. mineralische oder pflanzliche Öle, wirken sich bei der Blattapplikation sehr vorteilhaft aus.

Biologische Beispiele:

Beispiel 11:

Gametozide Aktivität bei kleinkörnigen Halmfrüchten

a) Induktion der männlichen Sterilität

Weizenpflanzen werden zu Beginn der Blütenbildung, d.h. etwa im 5½-Blattstadium mit einer aus Spritzpulver des Wirkstoffs hergestellten Spritzbrühe (3000 ppm Aktivsubstanz) gleichmässig besprüht. 2 bis 4 Wochen nach der Applikation, jedoch noch vor dem Antherenschieben, wird jede Ähre durch Abdecken gegen Fremdbestäubung geschützt. Die Bewertung der gametoziden Wirkung erfolgt zum Erntezeitpunkt durch Auszählen der gebildeten Körner pro Ähre. Als Vergleich dienen unbehandelte Weizenpflanzen.

b) Fruchtbarkeitstest (Bildung von Hybridsaatgut)

Eine Kontrollgruppe von Weizenpflanzen wird wie unter a) behandelt, abgedeckt oder isoliert gehalten und mit Blütenpollen einer anderen Weizensorte fremdbestäubt. Die Bewertung der Befruchtungsfähigkeit erfolgt zum Erntezeitpunkt durch Auszählen der gebildeten Hybrid-Körner pro Ähre. Als Vergleich dienen unbehandelte Weizenpflanzen und behandelte, aber abgedeckte Pflanzen.

Nitroaryl-alkylsulfonderivate aus den Tabellen 1 und 2 zeigen in obigen Versuchen a und b eine sehr gute gametozide Aktivität bei Weizen.

Die Verbindungen Nr. 1.1, 1.2, 1.5, 1.9, 1.10, 1.11, 1.14, 1.17, 1.19, 1.20, 1.23, 1.24, 1.28, 1.31, 1.32, 1.33, 1.34, 1.39, 1.41, 1.43, 1.44, 1.49, 1.52, 1.55, 1.59, 1.62, 1.67, 1.69, 1.73, 1.77–1.79, 2.1, 2.2 und 2.19–2.21 reduzieren die Bildung von Körnern pro Ähre auf 0 bis 15%. Selbst bei einer Wirkstoffkonzentration von 750 ppm unterdrücken unter anderem die Verbindungen Nr. 1.2, 1.5, 1.14, 1.24, 2.1 und 2.20 die Bildung von Körnern noch vollständig (unbehandelte Kontrolle = 100% Körner). Die mit den genannten Verbindungen behandelte zweite Kontrollgruppe (Test b) entwickelt sich nach der Fremdbestäubung wie die unbehandelten Pflanzen. Die Zahl der geernteten Hybrid-Körner beträgt 85 bis 100%. Bei den mit einer der Verbindungen Nr. 1.2, 1.5, 1.14, 1.24, 2.1 und 2.20 behandelten Weizenpflanzen liegt die Zahl der Hybrid-Körner sogar noch höher als bei der unbehandelten Vergleichsgruppe.

Vergleichbare Ergebnisse sind bei analogen Versuchen in Gerste und Roggen zu beobachten.

Beispiel 12:

Gametozide Aktivität bei Mais

a) Induktion der männlichen Sterilität

Maispflanzen werden bei der Blütenbildung, jedoch noch vor Sichtbarwerden des männlichen Blütenstandes mit einer Suspension des Wirkstoffs (3000 ppm Aktivsubstanz) gleichmässig besprüht. Kolben und Fahne der behandelten Pflanzen werden sorgfältig abgedeckt. Nach Ausbildung der männlichen Blütenteile, d.h. ca. 3 Wochen nach Applikation wird die gametozide Wirkung anhand der Fahnen- und Antherenausbildung beurteilt und die Pollensterilität anhand einer Selbstung überprüft. Dazu werden, sofern ausgebildet, Blütenpollen derselben Pflanze auf die Narben der Kolben übertragen (= Selbstung) und zum Zeitpunkt der Ernte die Maiskörner pro Kolben ausgezählt. Als Vergleich dient unbehandelter Mais.

b) Fruchtbarkeitstest (Bildung von Hybrid-saatgut)

Eine Kontrollgruppe von Maispflanzen wird wie unter a) behandelt und deren männliche und weibliche Blütenteile werden sorgfältig abgedeckt. Ca. 3 Wochen nach der Applikation werden die Kolben der behandelten Pflanzen mit Blütenpollen einer anderen, ausgewählten Maissorte fremdbestäubt. Zum Erntezeitpunkt wird die Fruchtbarkeit durch Auszählen der Hybrid-Körner pro Kolben beurteilt. Als Vergleich dient unbehandelter Mais.

In den obigen Versuchen a und b induzieren Verbindungen aus den Tabellen 1 und 2 eine nahezu vollständige männliche Sterilität. So führt z.B. die Behandlung mit den Verbindungen Nr. 1.1, 1.2, 1.5, 1.9, 1.14, 1.17, 1.19, 1.20, 1.24, 1.28, 1.31 bis 1.35, 1.41, 1.43, 1.44, 1.49, 1.55, 1.59, 1.62, 1.69, 1.77, 1.78, 1.79, 2.1, 2.2 und 2.19–2.21 zu einer Reduktion der Maiskörner pro Kolben auf weniger als 20%. Bei einer Wirkstoffkonzentration von 2000 ppm unterdrücken die Verbindungen Nr. 1.2, 1.5, 1.14, 1.24, 1.77, 2.1, 2.2, 2.19 und 2.20 die Entstehung der Körner noch vollständig (unbehandelte Kontrolle = 100% Körner). Bei keiner der Verbindungen wird die Fremdbestäubung merklich beeinträchtigt. Die Ausbeute an Hybrid-Körnern beträgt 85 bis 100%.

Beispiel 13:
I Gametozide Aktivität bei Sonnenblumen (Blattapplikation)

a) Induktion der männlichen Sterilität

Sonnenblumenpflanzen einer selbstfertilen Sorte werden ca. 3 Wochen vor Blühbeginn mit einer Emulsion des Wirkstoffs (3000 ppm Aktivsubstanz) gleichmässig besprüht. Die Infloreszenz-Knospen der behandelten Pflanzen werden zum Schutz vor Fremdbestäubung sorgfältig abgedeckt und zum Erntezeitpunkt die gametozide Wirkung durch Auszählen der noch gebildeten vollen Körner beurteilt. Als Vergleich dienen unbehandelte Sonnenblumen.

b) Fruchtbarkeitstest (Bildung von Hybrid-Saatgut)

Eine Kontrollgruppe von Sonnenblumenpflanzen wird wie unter a) behandelt. Die Knospen der behandelten Pflanzen werden sorgfältig abgedeckt. Nach dem Öffnen der Blüten wird eine Fremdbestäubung mit Blütenpollen einer anderen, ausgewählten Sonnenblumensorte durchgeführt. Dazu werden die intakten Blütenpollen in die Abdeckung eingebracht. Die Beurteilung der Fruchtbarkeit erfolgt zum Erntezeitpunkt durch Auszählen der gebildeten Hybrid-Körner. Als Vergleich dienen unbehandelte Sonnenblumen.

II Gametozide Aktivität bei Sonnenblumen (Bodenapplikation)

a) Induktion der männlichen Sterilität

Sonnenblumen einer selbstfertilen Sorte werden in Töpfen angepflanzt. 3–6 Wochen vor Blühbeginn wird die Erde in den Töpfen mit einer aus Spritzpulver des Wirkstoffs hergestellte Spritzbrühe (0,006% Aktivsubstanz bezogen auf das Erdvolumen) bewässert. Es wird dabei darauf geachtet, dass oberirdische Pflanzenteile unbenetzt bleiben. Die Knospen der behandelten Pflanzen werden sorgfältig abgedeckt und zum Erntezeitpunkt die gametozide Wirkung durch Auszählen der noch gebildeten vollen Körner im Vergleich zu unbehandelten Sonnenblumen beurteilt.

b) Fruchtbarkeitstest (Bildung von Hybrid-Saatgut)

Eine Kontrollgruppe von Sonnenblumenpflanzen wird wie unter a) angepflanzt und behandelt. Die Knospen der behandelten Pflanzen werden sorgfältig abgedeckt. Nach dem Öffnen der Blüte wird eine Fremdbestäubung mit Blütenpollen einer anderen, ausgewählten Sonnenblumensorte durchgeführt. Dazu werden die intakten Blütenpollen in die Abdeckung eingebracht. Die Beurteilung erfolgt zum Erntezeitpunkt durch Auszählen der gebildeten Hybrid-Körner. Als Vergleich dienen unbehandelte Sonnenblumen.

In obigen Versuchen I und II zeigen die Verbindungen aus den Tabellen 1 und 2 eine vergleichbare gametozide Aktivität. Nach Behandlung mit einem der Wirkstoffe Nr. 1.1, 1.2, 1.5, 1.9, 1.10, 1.11, 1.14, 1.17, 1.19, 1.24, 1.28, 1.31, 1.32, 1.33, 1.34, 1.35, 1.39, 1.41, 1.43, 1.44, 1.49, 1.52, 1.55, 1.59, 1.62, 1.67, 1.69, 1.73, 1.77–1.79, 2.1, 2.2 und 2.19–2.24 wird die Bildung von Samenkörnern im Vergleich zu unbehandelten Pflanzen auf weniger als 15% unterdrückt. Die Verbindungen Nr. 1.1, 1.2, 1.5, 1.14, 1.24, 1.77, 2.1, 2.2 und 2.19–2.24 erzielen sogar 100%ige männliche Sterilität, wobei die Fremdbefruchtung nicht merklich beeinflusst wird und zu einer Ausbeute an Hybrid-Körnern von 80 bis 100% führt.

Beispiel 14:
Blütenstimulation bei Baumwolle

Baumwollpflanzen werden vor Blühbeginn mit einer wässrigen Dispersion des Wirkstoffs (500 ppm Aktivsubstanz) gleichmässig besprüht und die Applikation, je nach Wachstumsrate, in Intervallen von 1 bis 3 Wochen wiederholt. Zum Erntezeitpunkt wird Kapsel- und Samenbildung im Vergleich zu unbehandelten Kontrollpflanzen beurteilt.

Verbindungen aus den Tabellen 1 und 2 zeigten bei Baumwolle eine ausgeprägte stimulierende Wirkung auf die Blütenbildung. Nach wiederholter Behandlung mit einer der Verbindungen Nr. 1.1, 1.2, 1.5, 1.9, 1.10, 1.11, 1.14, 1.17, 1.19, 1.28, 1.31, 1.32, 1.33, 1.34, 1.35, 1.39, 1.41, 1.43, 1.44, 1.49, 1.52, 1.55, 1.59, 1.62, 1.67, 1.69, 1.73, 1.77–1.79, 2.1, 2.2 und 2.19–2.21 wurde die Zahl der Blütenstände im Vergleich zu unbehandelten Pflanzen um 5 bis 25% erhöht. Die Pflanzen hatten ein gesundes Aussehen und zeigten eine qualitativ normale Kapsel- und Samenbildung.

Beispiel 15:
Gametozide Aktivität bei Tomaten

A) Induktion der männlichen Sterilität

Tomatenpflanzen werden vor Blühbeginn mit einer aus Spritzpulver hergestellten Spritzbrühe

des Wirkstoffs (1000 ppm Aktivsubstanz) gleichmässig besprüht und die Wirkstoff-Applikation, je nach Wachstumsrate der Pflanzen, in Intervallen wiederholt. Die Beurteilung der gametoziden Wirkung erfolgt zum Erntezeitpunkt durch Auszählen der Samen in den vollständig ausgebildeten Früchten pro Pflanze im Vergleich zu unbehandelten Tomatenpflanzen.

b) Fruchtbarkeitstest

Eine Gruppe von Kontrollpflanzen wird wie unter a) behandelt, jedoch während der Blüte mit ausgewählten Blütenpollen einer anderen Tomatensorte fremdbestäubt. Die Bewertung der Befruchtungsfähigkeit der behandelten Pflanzen erfolgte zum Erntezeitpunkt durch Auszählen der Samen in den vollständig ausgebildeten Früchten pro Pflanze und im Vergleich zu unbehandelten Tomatenpflanzen.

In obigem Versuch zeigen Verbindungen aus den Tabellen 1 und 2 sehr gute gametozide Eigenschaften in Tomatenkulturen. Nach Behandlung mit einem der Wirkstoffe Nr. 1.1, 1.2, 1.5, 1.9, 1.10, 1.11, 1.14, 1.17, 1.19, 1.24, 1.28, 1.31, 1.32, 1.33, 1.34, 1.35, 1.39, 1.41, 1.43, 1.44, 1.49, 1.52, 1.55, 1.59, 1.62, 1.67, 1.69, 1.73, 1.77–1.79, 2.1, 2.2, 2.19, 2.20, 2.21 und 2.22 wird die Fruchtgrösse im Vergleich zu unbehandelten Pflanzen reduziert, dabei zeigt es sich, dass die Früchte weniger oder keine Samen enthalten. Bei Behandlung mit einer der Verbindungen Nr. 1.1, 1.2, 1.5, 1.14, 1.24, 1.77 und 2.1 erfolgt eine vollständige männliche Sterilisierung. Die Fremdbefruchtung wird nicht merklich beeinflusst, ebenso bei Verbindung 2.19 und 2.20.

Beispiel 16:
Gametozide Aktivität bei Leguminosen

a) Induktion der männlichen Sterilität (postemergente Behandlung)

Buschbohnen der Sorte «Miry» werden im Knospenstadium der Blüten mit einer Suspension des Wirkstoffs (2000 ppm Aktivsubstanz) gleichmässig besprüht und die Knospen anschliessend sorgfältig abgedeckt. Nach Ausbildung der Blüten wird die Pollenbildung beurteilt und die Pollensterilität durch Kreuzung mit ausgewählten, von Hand sterilisierten Blüten unbehandelter Kontrollpflanzen geprüft. Als Kontrolle werden analoge Kreuzungen auf unbehandelten Pflanzen durchgeführt. In einem parallelen Versuch wird die Blattapplikation durch eine Drenchapplikation (= Angiessen der Wurzelzone mit 4 kg/AS/ha) ersetzt.

b) Fruchtbarkeit

Eine Kontrollgruppe von Buschbohnen wird wie unter a) behandelt und die Blütenknospen sorgfältig abgedeckt. Nach dem Öffnen werden die Blüten mit Pollen von unbehandelten Blüten bestäubt und die Befruchtungsrate mit derjenigen von kreuzbestäubten, unbehandelten, handsterilisierten Blüten aus Versuch a) verglichen.

In obigen Versuchen bewirkten die Verbindungen Nr. 1.1, 1.2, 1.5, 1.9, 1.10, 1.11, 1.14, 1.17, 1.19, 1.20, 1.23, 1.24, 1.27, 1.28, 1.31–1.34, 1.39, 1.40, 1.41, 1.43, 1.44, 1.49, 1.52, 1.55, 1.59, 1.67, 1.69, 1.73, 1.77–1.79, 2.1, 2.20 und 2.21 eine Pollenreduktion um 85 bis 90% und eine Pollenstabilität von 90 bis 100% im Vergleich zu unbehandelten Kontrollpflanzen, deren Pollen voll ausgebildet und intakt waren. Bei den behandelten Pflanzen wurde keine merkliche Beeinflussung der weiblichen Blütenteile bei der Befruchtung mit unbehandelten Fremdpollen (95–100% Fruchtbildung) beobachtet, jedoch entwickelten sich zum Teil parthenocarpe Früchte.

Beispiel 17:
Gametozide Aktivität bei Lein

a) Induktion der männlichen Sterilität

Lein der Sorte «Blaublühender» wird im Knospenstadium mit einer Suspension des Wirkstoffs (500 und 100 ppm Aktivsubstanz) gleichmässig besprüht und die Knospen anschliessend sorgfältig abgedeckt. Nach Ausbildung der Blüten wird die Pollenbildung beurteilt und die Pollensterilität durch Kreuzung mit ausgewählten, von Hand sterilisierten Blüten unbehandelter Kontrollpflanzen geprüft. Als Vergleich werden analoge Kreuzungen auf unbehandelten Pflanzen durchgeführt.

In einem parallelen Versuch wird die Blattapplikation durch eine Drenchapplikation (= Angiessen der Wurzelzone mit 3 kg/AS/ha) ersetzt.

b) Fruchtbarkeitstest

Eine Kontrollgruppe von Leinpflanzen wird wie unter a) behandelt und die Blütenknospen sorgfältig abgedeckt. Nach dem Öffnen werden die Blüten mit Pollen von unbehandelten Blüten bestäubt und die Befruchtungsrate mit derjenigen von kreuzbestäubten, unbehandelten, handsterilisierten Blüten aus Versuch a) verglichen.

In obigen Versuchen bewirkten die Verbindungen Nr. 1.1, 1.2, 1.5, 1.9, 1.10, 1.11, 1.17, 1.20, 1.23, 1.24, 1.27, 1.28, 1.31–1.34, 1.39, 1.40, 1.41, 1.43, 1.44, 1.49, 1.52, 1.55, 1.59, 1.67, 1.69, 1.73, 1.77–1.79, 2.1, 2.2 und 2.19–2.21 eine Pollenreduktion von etwa 90% und eine fast vollständige Sterilität (95 bis 100%) der Pollen im Vergleich zu unbehandelten Kontrollpflanzen, bei denen die Pollen voll ausgebildet und befruchtungsfähig waren. Bei den behandelten Pflanzen wurde keine merkliche Beeinflussung der weiblichen Blütenteile bei der Befruchtung mit unbehandelten Fremdpollen beobachtet (95–100% Fruchtbildung), jedoch entwickelten sich zum Teil parthenocarpe Früchte.

Beispiel 18:
Wirkung gegen Cercospora arachidicola auf Erdnusspflanzen

a) Residual-protektive Wirkung

10–15 cm hohe Erdnusspflanzen wurden mit einer aus Spritzpulver der Wirksubstanz hergestellten Spritzbrühe (0,02% Aktivsubstanz) besprüht und 48 Stunden später mit einer Konidiensuspension des Pilzes infiziert. Die infizierten Pflanzen wurden während 72 Stunden bei ca. 21 °C und hoher Luftfeuchtigkeit inkubiert und anschliessend bis zum Auftreten der typischen Blattflecken in einem Gewächshaus aufgestellt. Die Beurteilung der fungiziden Wirkung erfolgte 12 Tage nach der Infektion basierend auf Anzahl und Grösse der auftretenden Flecken.

b) Systemische Wirkung

Zu 10–15 cm hohen Erdnusspflanzen wurde eine aus Spritzpulver des Wirkstoffes hergestellte Spritzbrühe gegossen (0,006% Aktivsubstanz, bezogen auf das Erdvolumen). Nach 48 Stunden wurden die behandelten Pflanzen mit einer Konidiensuspension des Pilzes infiziert und 72 Stunden bei ca. 21 °C und hoher Luftfeuchtigkeit inkubiert. Anschliessend wurden die Pflanzen im Gewächshaus aufgestellt und nach 11 Tagen der Pilzbefall beurteilt.

Im Vergleich zu unbehandelten, aber infizierten Kontrollpflanzen (Anzahl und Grösse der Flecken = 100%), zeigten Erdnusspflanzen, die mit Wirkstoffen aus den Tabellen 1 oder 2 behandelt wurden, einen stark reduzierten Cercosporabefall. So verhinderten die Verbindungen Nr. 1.1, 1.2, 1.24, 1.31, 1.35, 1.43, 1.79, 1.80, 2.1 und 2.2 in obigen Versuchen das Auftreten von Flecken fast vollständig (0–10%).

Beispiel 19:
Wirkung gegen Puccinia graminis auf Weizen
a) Residual-protektive Wirkung

Weizenpflanzen wurden 6 Tage nach der Aussaat mit einer aus Spritzpulver des Wirkstoffes hergestellten Spritzbrühe (0,06% Aktivsubstanz) besprüht. Nach 24 Stunden wurden die behandelten Pflanzen mit einer Uredosporensuspension des Pilzes infiziert. Nach einer Inkubation während 48 Stunden bei 95–100% relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

b) Systemische Wirkung

Zu Weizenpflanzen wurde 5 Tage nach der Aussaat eine aus Spritzpulver des Wirkstoffes hergestellte Spritzbrühe gegossen (0,06% Aktivsubstanz, bezogen auf das Bodenvolumen). Nach 48 Stunden wurden die behandelten Pflanzen mit einer Uredosporensuspension des Pilzes infiziert. Nach einer Inkubation während 48 Stunden bei 95–100% relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

Verbindungen der Formel I zeigten gegen Puccinia-Pilze eine gute Wirkung. Unbehandelte aber infizierte Kontrollpflanzen zeigten einen Pucciniabefall von 100%. Unter anderen hemmten die Verbindungen Nr. 1.33, 1.34, 1.35, 1.78, 1.79 und 1.80 den Pilzbefall auf 0 bis 5%.

Beispiel 20:
Wirkung gegen Erysiphe graminis auf Gerste
a) Residual-protektive Wirkung

Ca. 8 cm hohe Gerstenpflanzen wurden mit einer aus Spritzpulver des Wirkstoffes hergestellten Spritzbrühe (0,02% Aktivsubstanz) besprüht. Nach 3–4 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,006% Aktivsubstanz, bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

Verbindungen der Formel I zeigten eine gute Wirkung gegen Erysiphe-Pilze. Unbehandelte, aber infizierte Kontrollpflanzen zeigten einen Erysiphe-Befall von 100%. Unter anderen hemmten die Verbindungen Nr. 1.1, 1.2, 1.24, 1.33, 1.34, 1.78, 1.79 und 1.80 den Pilzbefall bei Gerste auf weniger als 10%.

Beispiel 21:
Wirkung gegen Xanthomonas oryzae auf Reis
a) Residual-protektive Wirkung

Reispflanzen der Sorte «Caloro» oder «S6» wurden nach 3-wöchiger Anzucht im Gewächshaus mit der Prüfsubstanz in Form einer Spritzbrühe (0,06% Aktivsubstanz) besprüht. Nach eintägigem Antrocknen dieses Spritzbelages wurden die Pflanzen in einem Klimaraum bei 24 °C und 75–85% relativer Luftfeuchtigkeit aufgestellt und infiziert. Die Infektion erfolgte, indem die Blattspitzen mit einer Schere, die zuvor in eine Suspension von Xanthomonas oryzae eingetaucht worden war, abgeschnitten wurden. Nach 10-tägiger Inkubation in demselben Raum wurden die angeschnittenen Blätter welk, rollten sich ein und wurden nekrotisch. Das Ausmass dieser Krankheitssymptome diente zur Beurteilung der residualen Wirksamkeit der Prüfsubstanz.

b) Systemische Wirkung

Reispflanzen der Sorte «Caloro» oder «S6» wurden nach 3-wöchiger Anzucht im Gewächshaus mit einer Suspension der Prüfsubstanz begossen (0,006% Aktivsubstanz, bezogen auf das Erdvolumen). Drei Tage nach dieser Behandlung wurden die Pflanzen in einem Klimaraum bei 24 °C und 75–85% relativer Luftfeuchtigkeit aufgestellt und infiziert. Die Infektion erfolgte, indem die Blattspitzen mit einer Schere, die zuvor in eine Suspension von Xanthomonas oryzae eingetaucht worden war, abgeschnitten wurden. Nach 10-tägiger Inkubation in demselben Raum wurden die angeschnittenen Blätter welk, rollten sich ein und wurden nekrotisch. Das Ausmass dieser Krankheitssymptome diente zur Beurteilung der systemischen Wirksamkeit der Prüfsubstanz.

Verbindungen aus den Tabellen 1 und 2 zeigten eine gute Wirkung gegen Xanthomonas-Bakterien so hemmten bei der residual-protektiven Behandlung von Reis unter anderen die Verbindungen Nr. 1.1, 1.2, 1.5, 1.9, 1.10, 1.11, 1.14, 1.19, 1.20, 1.23, 1.24, 1.28, 1.31, 1.32, 1.34, 1.35, 1.39, 1.41, 1.43, 1.44, 1.49, 1.52, 1.59, 1.69, 1.73, 1.77, 2.1 und 2.2 das Auftreten von nekrotischen Flecken fast vollständig (0 bis 5%). Darüber hinaus zeigten die Verbindungen Nr. 1.1, 1.2, 1.5, 1.9, 1.10, 1.11, 1.19, 1.20, 1.23, 1.24, 1.28, 1.31, 1.32, 1.34, 1.39, 1.41, 1.43, 1.49, 1.52, 1.59, 1.69, 1.73, 2.1 und 2.2 auch bei systemischem Einsatz ihre volle Wirkung (0 bis 5% Nekrose). Unbehandelte aber infizierte Reispflanzen (Kontrolle) wiesen eine Nekrose von 100% auf.

Beispiel 22:
Wirkung gegen Xanthomonas vesicatoria auf Paprika

a) Residual-protektive Wirkung

Paprikapflanzen der Sorte «California Wonder» wurden nach 3-wöchiger Anzucht im Gewächshaus mit der Prüfsubstanz in Form einer Spritzbrühe (0,06% Aktivsubstanz) besprüht. Nach eintägigem Antrocknen dieses Spritzbelages wurden die Pflanzen in einem Klimaraum bei 26 °C und 95–100% relativer Luftfeuchtigkeit aufgestellt und durch Besprühen der Blattunterseiten mit einer standardisierten Suspension von Xanthomonas vesicatoria infiziert. Nach 6-tägiger Inkubation in demselben Raum entstanden auf den Blättern runde, anfangs wässrige, später nekrotische, aufgehellte Flecken. Das Ausmass dieser Flecken diente zur Beurteilung der residualen Wirksamkeit der Prüfsubstanz.

b) Systemische Wirkung

Paprikapflanzen der Sorte «California Wonder» wurden nach 3-wöchiger Anzucht im Gewächshaus mit einer Suspension der Prüfsubstanz begossen (0,006% Aktivsubstanz, bezogen auf das Erdvolumen). Drei Tage nach dieser Behandlung wurden die Pflanzen in einem Klimaraum bei 26 °C und 95–100% relativer Luftfeuchtigkeit aufgestellt und durch Besprühen der Blattunterseiten mit einer standardisierten Suspension von Xanthomonas vesicatoria infiziert. Nach 6-tägiger Inkubation in demselben Raum entstanden auf den Blättern runde, anfangs wässrige, später nekrotische, aufgehellte Flecken. Das Ausmass dieser Flecken diente zur Beurteilung der systemischen Wirksamkeit der Prüfsubstanz. Verbindungen aus beiden Tabellen verhinderten das Auftreten von Flecken fast vollständig.

**Patentansprüche**

1. Verwendung einer Verbindung der Formel I

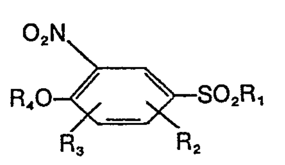

(I),

worin

$R_1$ $C_1$–$C_6$-Alkyl bedeutet;

$R_2$ und $R_3$ unabhängig voneinander für Wasserstoff, $C_1$–$C_6$-Alkyl, $C_1$–$C_6$-Alkoxy, $C_1$–$C_6$-Haloalkyl, Halogen, Cyano, Nitro, Amino stehen oder orthoständig zueinander als Gruppe $-(CH=CH)_2-$ einen Naphthalinring vervollständigen;

$R_4$ für Wasserstoff, ein Kation oder für die Gruppe $-COR_5$ steht,

wobei

$R_5$ $C_1$–$C_{12}$-Alkyl, $C_1$–$C_{12}$-Alkoxy, $C_1$–$C_{12}$-Haloalkoxy, $C_2$–$C_{10}$-Alkenyl, $C_2$–$C_{10}$-Haloalkenyl, $C_2$–$C_6$-Alkinyl, $C_3$–$C_7$-Cycloalkyl, Phenyl, Benzyl, Furyl oder Tetrahydrofuryl bedeutet, und wobei jeder der cyclischen Substituenten unsubstituiert oder ein- oder mehrfach durch gleiche oder verschiedene Substituenten aus der Reihe $C_1$–$C_3$-Alkyl, $C_1$–$C_3$-Alkoxy, $C_1$–$C_3$-Haloalkyl, Halogen, Cyano und Nitro substituiert ist, als Wirkstoffe zur Stimulierung des generativen Pflanzenwuchses.

2. Verwendung gemäss Anspruch 1 einer Verbindung der Formel I, worin $R_1$ für $C_1$–$C_3$-Alkyl steht, $R_2$ und $R_3$ unabhängig voneinander für Wasserstoff, $C_1$–$C_3$-Alkyl, $C_1$–$C_3$-Alkoxy, $C_1$–$C_3$-Haloalkyl, Halogen, Cyano, Nitro, Amino stehen oder orthoständig zueinander als Gruppe $-(CH=CH)-$ einen Naphthalinring vervollständigen; $R_4$ für Wasserstoff, ein Alkali- oder Erdalkalikation oder für die Gruppe $-COR_5$ steht, wobei $R_5$ $C_1$–$C_6$-Alkyl, $C_1$–$C_6$-Alkoxy, $C_1$–$C_6$-Haloalkoxy, $C_2$–$C_5$-Alkenyl, $C_2$–$C_5$-Haloalkenyl, $C_2$–$C_5$-Alkinyl, $C_3$–$C_7$-Cycloalkyl, Phenyl, Benzyl, Furyl oder Tetrahydrofuryl bedeutet, und wobei jeder der cyclischen Substituenten unsubstituiert oder durch gleiche oder verschiedene Substituenten aus der Reihe Methyl, Ethyl, Methoxy, Ethoxy, Halomethyl, Fluor, Chlor, Brom, Cyano und Nitro substituiert ist.

3. Verwendung gemäss Anspruch 1 oder 2 einer Verbindung der Formel I, worin $R_1$ für Methyl oder Ethyl steht, $R_2$ Wasserstoff bedeutet, $R_3$ für Wasserstoff, Methyl, Methoxy, Trifluormethyl, Brom, Cyano, Nitro oder Amino steht,

$R_4$ Wasserstoff oder die Gruppe $-COR_5$ repräsentiert, wobei $R_5$ $C_1$–$C_3$-Alkyl, $C_1$–$C_3$-Alkoxy, $C_1$–$C_3$-Haloalkoxy, $C_2$–$C_5$-Alkenyl, $C_2$–$C_5$-Haloalkenyl, $C_2$–$C_5$-Alkinyl, $C_3$–$C_7$-Cycloalkyl, Phenyl, Benzyl, Furyl oder Tetrahydrofuryl bedeutet.

4. Verwendung gemäss einem der Ansprüche 1 bis 3 einer Verbindung der Formel I, worin $R_1$ für Methyl steht, $R_2$ und $R_3$ Wasserstoff bedeuten und $R_4$ die Gruppe $-COR_5$ repräsentiert, wobei $R_5$ $C_1$–$C_3$-Alkyl, $C_1$–$C_3$-Alkoxy, $C_2$–$C_5$-Alkenyl, $C_2$–$C_5$-Haloalkenyl, $C_3$–$C_7$-Cycloalkyl, Phenyl, Benzyl, Furyl oder Tetrahydrofuryl bedeutet.

5. Verwendung gemäss Anspruch 1 eines Hydrazinium- oder Ammoniumsalzes der Formel I.

6. Verwendung gemäss Anspruch 1 oder 5 eines Ammoniumsalzes der Formel I, worin das Kation die Formel $NH_{(4-a)}(Niederalkyl)_a^+$ aufweist, wobei a eine der Zahlen 1, 2, 3 oder 4 repräsentiert.

7. Verwendung gemäss einem der Ansprüche 1 bis 6, wobei der Wirkstoff der Formel I eine der Verbindungen

4-Hydroxy-3-nitrophenyl-methylsulfon,

4-[3-chlor-n-propylcarbonyloxy]-3-nitrophenyl-methylsulfon,
4-Hydroxy-3-nitrophenyl-methylsulfon-tetra(n-butyl)ammoniumsalz,
4-Acetyloxy-3-nitrophenyl-methylsulfon,
4-Cyclopropylcarbonyl-3-nitrophenyl-methylsulfon,
4-Hydroxy-3-nitrophenyl-ethylsulfon,
4-Acryloxy-3-nitrophenyl-methylsulfon,
4-Trichloracryloxy-3-nitrophenyl-methylsulfon,
4-n-Propylcarbonyloxy-3-nitrophenyl-methylsulfon,
4-Ethylcarbonyloxy-3-nitrophenyl-methylsulfon,
4-Phenylcarbonyloxy-3-nitrophenyl-methylsulfon,
4-Methoxymethylcarbonyloxy-3-nitrophenyl-methylsulfon,
4-Cyclohexylcarbonyloxy-3-nitrophenyl-methylsulfon,
4-Chlormethylcarbonyloxy-3-nitrophenyl-methylsulfon oder
4-Methoxycarbonyloxy-3-nitrophenyl-methylsulfon
ist.

8. Verwendung gemäss einem der Ansprüche 1 bis 7, wobei die Stimulierung des generativen Pflanzenwuchses in der Stimulierung der Blütenbildung besteht.

9. Verwendung gemäss Anspruch 8, wobei die Stimulierung der Blütenbildung zur Erzeugung männlich steriler, jedoch fortpflanzungsfähiger Pflanzen führt.

10. Verwendung gemäss einem der Ansprüche 1 bis 9, wobei die Pflanzen, deren generatives Wachstum stimuliert wird, Kulturpflanzen sind.

11. Verwendung gemäss Anspruch 10, wobei es sich bei den Kulturpflanzen um kleinkörnige Halmfrucht- oder Getreidepflanzen oder um Futtergräser handelt.

12. Verwendung gemäss Anspruch 10, wobei es sich bei den Kulturpflanzen um Weizen, Roggen, Gerste, Hafer, Reis, Mais, Sorghum-Hirse, Lein, Avocados, Leguminosen, Sonnenblumen, Baumwolle, Gemüse oder Zierpflanzen handelt.

13. Verwendung gemäss einem der Ansprüche 1 bis 12, wobei der Wirkstoff der Formel I in einer Aufwandmenge von 0,05 bis 12 kg pro ha appliziert wird.

14. Verwendung gemäss Anspruch 13, wobei die Aufwandmenge 0,5 bis 8 kg pro ha beträgt.

15. Verwendung gemäss Anspruch 13 oder 14, wobei die Aufwandmenge 1 bis 4 kg pro ha beträgt.

16. Verwendung gemäss einem der Ansprüche 10 bis 12, wobei die Applikation des Wirkstoffes der Formel I nach dem Auflaufen der Pflanzen, jedoch vor dem Auftreten der Ähren oder Antheren erfolgt.

17. Verwendung gemäss Anspruch 16, wobei die Applikation im 5½ Blattstadium erfolgt.

18. Verwendung gemäss Anspruch 17, wobei die Applikation vor Blühbeginn erfolgt.

19. Verfahren zur Gewinnung von Hybrid-Samen, dadurch gekennzeichnet, dass man eine als weibliche Elternpflanze ausgewählte Stammpflanze zu Beginn der Blütenbildung, aber vor Ausbildung der männlichen Blütenteile durch Applikation einer Verbindung der Formel I gemäss Anspruch 1, männlich sterilisiert und mit Pollen einer ausgewählten genetisch-ähnlichen Pflanze fremdbestäubt und den reifen Hybrid-Samen erntet.

20. Verfahren nach Anspruch 19, dadurch gekennzeichnet, dass man den Wirkstoff in einer Aufwandmenge von 0,05 bis 12 kg Aktivsubstanz pro ha auf die weibliche Elternpflanze oder deren Standort appliziert.

## Claims

1. Use of a compound of the formula I

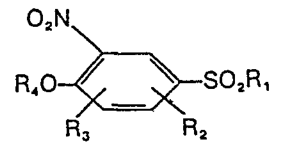

(I)

wherein

$R_1$ is $C_1$–$C_6$alkyl,

$R_2$ and $R_3$, each independently of the other, are hydrogen, $C_1$–$C_6$alkyl, $C_1$–$C_6$alkoxy, $C_1$–$C_6$haloalkyl, halogen, cyano, nitro or amino, or both together in the ortho-position to each other as a $-(CH=CH)_2-$ group complete a naphthalene ring,

$R_4$ is hydrogen, a cation or the $-COR_5$ group, wherein $R_5$ is $C_1$–$C_{12}$alkyl, $C_1$–$C_{12}$alkoxy, $C_1$–$C_{12}$ haloalkoxy, $C_2$–$C_{10}$alkenyl, $C_2$–$C_{10}$haloalkenyl, $C_2$–$C_6$alkynyl, $C_3$–$C_7$cycloalkyl, phenyl, benzyl, furyl or tetrahydrofuryl, and where each of the cyclic substituents is unsubstituted or mono- or polysubstituted by the same or different substituents selected from the group consisting of $C_1$–$C_3$alkyl, $C_1$–$C_3$alkoxy, $C_1$–$C_3$haloalkyl, halogen, cyano and nitro,

as an active component for the stimulation of generative plant growth.

2. Use according to claim 1 of a compound of the formula I, wherein $R_1$ is $C_1$–$C_3$alkyl, $R_2$ and $R_3$, each independently of the other, are hydrogen, $C_1$–$C_3$alkyl, $C_1$–$C_3$alkoxy, $C_1$–$C_3$haloalkyl, halogen, cyano, nitro or amino, or both together in the ortho-position to each other as $-(CH=CH)_2-$ group complete a naphthalene ring; $R_4$ is hydrogen, an alkali metal cation or alkaline earth metal cation or the $-COR_5$ group, wherein $R_5$ is $C_1$–$C_6$alkyl, $C_1$–$C_6$alkoxy, $C_1$–$C_6$haloalkoxy, $C_2$–$C_5$alkenyl, $C_2$–$C_5$haloalkenyl, $C_2$–$C_5$alkynyl, $C_3$–$C_7$cycloalkyl, phenyl, benzyl, furyl or tetrahydrofuryl, and wherein each of the cyclic substituents is unsubstituted or substituted by identical or different substituents selected from the group consisting of methyl, ethyl, methoxy, ethoxy, halomethyl, fluorine, chlorine, bromine, cyano and nitro.

3. Use according to either of claims 1 or 2 of a compound of the formula I, in which $R_1$ is methyl or ethyl, $R_2$ is hydrogen, $R_3$ is hydrogen, methyl, methoxy, trifluoromethyl, bromine, cyano, nitro or amino, $R_4$ is hydrogen, or the $-COR_5$ group, wherein $R_5$ is $C_1$–$C_3$alkyl, $C_1$–$C_3$alkoxy, $C_1$–$C_3$haloalkoxy, $C_2$–$C_5$alkenyl, $C_2$–$C_5$haloalkenyl,

$C_2$–$C_5$alkynyl, $C_3$–$C_7$cycloalkyl, phenyl, benzyl, furyl or tetrahydrofuryl.

4. Use according to any one of claims 1 to 3 of a compound of the formula I, in which $R_1$ is methyl, $R_2$ and $R_3$ are hydrogen and $R_4$ is hydrogen or the $-COR_5$ group, wherein $R_5$ is $C_1$–$C_3$alkyl, $C_1$–$C_3$alkoxy, $C_2$–$C_5$alkenyl, $C_2$–$C_5$haloalkenyl, $C_3$–$C_7$cycloalkyl, phenyl, benzyl, furyl or tetrahydrofuryl.

5. Use according to claim 1 of a hydrazinium or ammonium salt of the formula I.

6. Use according to either of claims 1 or 5 of an ammonium salt of the formula I whose cation has the formula $NH_{(4-a)}(\text{lower alkyl})_a^+$, wherein a is 1, 2, 3 or 4.

7. Use according to any one of claims 1 to 6, wherein the active component of the formula I is one of the compounds
4-hydroxy-3-nitrophenylmethylsulfone,
4-[3-chloro-n-propylcarbonyloxy]-3-nitrophenylmethylsulfone,
4-hydroxy-3-nitrophenylmethylsulfone-tetra(n-butyl)ammonium salt,
4-acetyloxy-3-nitrophenylmethylsulfone,
4-cyclopropylcarbonyl-3-nitrophenylmethylsulfone,
4-hydroxy-3-nitrophenylethylsulfone,
4-acryloxy-3-nitrophenylmethylsulfone,
4-trichloroacryloxy-3-nitrophenylmethylsulfone,
4-n-propylcarbonyloxy-3-nitrophenylmethylsulfone,
4-ethylcarbonyloxy-3-nitrophenylmethylsulfone,
4-phenylcarbonyloxy-3-nitrophenylmethylsulfone,
4-methoxymethylcarbonyloxy-3-nitrophenylmethylsulfone,
4-cyclohexylcarbonyloxy-3-nitrophenylmethylsulfone,
4-chloromethylcarbonyloxy-3-nitrophenylmethylsulfone or
4-methoxycarbonyloxy-3-nitrophenylmethylsulfone.

8. Use according to any one of claims 1 to 7, wherein the stimulation of the generative plant growth consists in the stimulation of flower formation.

9. Use according to claim 8, wherein the stimulation of flower formation results in obtaining male-sterile plants which are capable of reproduction.

10. Use according to any one of claims 1 to 9, wherein the plants whose generative growth is stimulated are cultivated plants.

11. Use according to claim 10, wherein the cultivated plants are small grain plants, cereals or forage grasses.

12. Use according to claim 10, wherein the cultivated plants are wheat, rye, barley, oats, rice, maize, sorghum, flax, avocados, leguminosae, sunflowers, cotton, vegetables or ornamentals.

13. Use according to any one of claims 1 to 12, wherein the compound of formula I is applied at a rate of application of 0.05 to 12 kg of active ingredient per hectare.

14. Use according to claim 13, wherein the rate of application is in the range from 0.5 to 8 kg per hectare.

15. Use according to either of claims 13 or 14, wherein the rate of application is in the range from 1 to 4 kg per hectare.

16. Use according to any one of claims 10 to 12, wherein application of the compound of formula I is made postemergence, but before ears and anthers have appeared.

17. Use according to claim 16, wherein application is made in the 5½-leaf stage.

18. Use according to claim 17, wherein application is made before the start of flowering.

19. A method of obtaining hybrid seeds, which comprises inducing male sterilisation in a seed plant selected as female parent plant at the start of flower formation but before formation of the male flower parts, by applying a compound of the formula I according to claim 1, cross-pollinating said plant with pollen of a selected genetically similar plant, and harvesting the ripe hybrid seeds.

20. A method according to claim 19, wherein the compound of formula I is applied at a rate of application of 0.05 to 12 kg of active ingredient per hectare to the female parent plant or to the locus thereof.

**Revendications**

1. Utilisation d'un composé de formule I

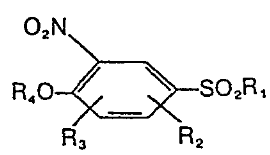

(I)

dans laquelle
$R_1$ représente un groupe alkyle en C 1–C 6;
$R_2$ et $R_3$ représentent chacun, indépendamment l'un de l'autre, l'hydrogène, un groupe alkyle en C 1–C 6, alcoxy en C 1–C 6, halogénoalkyle en C 1–C 6, un halogène, un groupe cyano, nitro, amino ou bien, en position mutuelle ortho, forment ensemble un groupe $-(CH=CH)_2-$ qui complète un cycle naphtalénique;
$R_4$ représente l'hydrogène, un cation ou le groupe $-COR_5$ dans lequel
$R_5$ représente un groupe alkyle en C 1–C 12, alcoxy en C 1–C 12, halogénoalcoxy en C 1–C 12, alcényle en C 2–C 10, halogénoalcényle en C 2–C 10, alcynyle en C 2–C 6, cycloalkyle en C 3–C 7, phényle, benzyle, furyle ou tétrahydrofuryle, chacun des substituants cycliques étant non substitué ou portant un ou plusieurs substituants identiques ou différents choisis parmi les groupes alkyle en C 1–C 3, alcoxy en C 1–C 3, halogénoalkyle en C 1–C 3, les halogènes, les groupes cyano et nitro, en tant que substances actives servant à stimuler la croissance générative des plantes.

2. Utilisation selon la rev. 1, d'un composé de formule I dans laquelle $R_1$ représente un groupe alkyle en C 1–C 3, $R_2$ et $R_3$ représentent chacun,

indépendamment l'un de l'autre, l'hydrogène, un groupe alkyle en C 1–C 3, alcoxy en C 1–C 3, halogénoalkyle en C 1–C 3, un halogène, un groupe cyano, nitro, amino ou bien, en position mutuelle ortho, forment ensemble un groupe –(CH=CH)– qui complète un cycle naphtalénique; $R_4$ représente l'hydrogène, un cation alcalin ou alcalino-terreux ou le groupe $-COR_5$ dans lequel $R_5$ représente un groupe alkyle en C 1–C 6, alcoxy en C 1–C 6, halogénoalcoxy en C 1–C 6, alcényle en C 2–C 5, halogénoalcényle en C 2–C 5, alcynyle en C 2–C 5, cycloalkyle en C 3–C 7, phényle, benzyle, furyle ou tétrahydrofuryle, chacun des substituants cycliques étant non substitué ou portant des substituants identiques ou différents choisis parmi les groupes méthyle, éthyle, méthoxy, éthoxy, halogénométhyle, le fluor, le chlore, le brome, les groupes cyano et nitro.

3. Utilisation selon la rev. 1 ou 2, d'un composé de formule I dans laquelle $R_1$ représente un groupe méthyle ou éthyle, $R_2$ représente l'hydrogène, $R_3$ représente l'hydrogène, un groupe méthyle, méthoxy, trifluorométhyle, le brome, un groupe cyano, nitro ou amino, $R_4$ représente l'hydrogène ou le groupe $-COR_5$ dans lequel $R_5$ représente un groupe alkyle en C 1–C 3, alcoxy en C 1–C 3, halogénoalcoxy en C 1–C 3, alcényle en C 2–C 5, halogénoalcényle en C 2–C 5, alcynyle en C 2–C 5, cycloalkyle en C 3–C 7, phényle, benzyle, furyle ou tétrahydrofuryle.

4. Utilisation selon l'une des rev. 1 à 3, d'un composé de formule I dans laquelle $R_1$ représente un groupe méthyle, $R_2$ et $R_3$ représentent l'hydrogène et $R_4$ le groupe $-COR_5$ dans lequel $R_5$ représente un groupe alkyle en C 1–C 3, alcoxy en C 1–C 3, alcényle en C 2–C 5, halogénoalcényle en C 2–C 5, cycloalkyle en C 3–C 7, phényle, benzyle, furyle ou tétrahydrofuryle.

5. Utilisation selon la rev. 1, d'un sel d'hydrazinium ou d'ammonium de formule I.

6. Utilisation selon la rev. 1 ou 5, d'un sel d'ammonium de formule I dans lequel le cation répond à la formule $NH_{(4-a)}(\text{alkyle inférieur})_a^+$ dans laquelle a est égal à 1, 2, 3 ou 4.

7. Utilisation selon l'une des rev. 1 à 6, de la substance active de formule I consistant en l'un des composés suivants:
4-hydroxy-3-nitrophényl-méthylsulfone,
4-[3-chloro-n-propylcarbonyloxy]-3-nitrophényl-méthylsulfone,
sel de tétra-(n-butyl)-ammonium de la 4-hydroxy-3-nitrophényl-méthylsulfone,
4-acétyloxy-3-nitrophényl-méthylsulfone,
4-cyclopropylcarbonyl-3-nitrophényl-méthylsulfone,
4-hydroxy-3-nitrophényl-éthylsulfone,
4-acryloxy-3-nitrophényl-méthylsulfone,
4-trichloracryloxy-3-nitrophényl-méthylsulfone,
4-n-propylcarbonyloxy-3-nitrophényl-méthylsulfone,
4-éthylcarbonyloxy-3-nitrophényl-méthylsulfone,
4-phénylcarbonyloxy-3-nitrophényl-méthylsulfone,
4-méthoxyméthylcarbonyloxy-3-nitrophényl-méthylsulfone,
4-cyclohexylcarbonyloxy-3-nitrophényl-méthylsulfone,
4-chlorométhylcarbonyloxy-3-nitrophényl-méthylsulfone, ou
4-méthoxycarbonyloxy-3-nitrophényl-méthylsulfone.

8. Utilisation selon l'une des rev. 1 à 7, dans laquelle la stimulation de la croissance générative des plantes consiste en la stimulation de la floraison.

9. Utilisation selon la rev. 8, dans laquelle la stimulation de la floraison conduit à une formation de plantes à organes mâles stériles, mais aptes à la multiplication.

10. Utilisation selon l'une des rev. 1 à 9, dans laquelle les plantes dont la croissance générative est stimulée sont des plantes cultivées.

11. Utilisation selon la rev. 10, dans laquelle les plantes cultivées sont des céréales à petites graines ou des graminées fourragères.

12. Utilisation selon la rev. 10, dans laquelle les plantes cultivées sont le blé, le seigle, l'orge, l'avoine, le riz, le maïs, le millet-sorgho, le lin, l'avocat, des légumineuses, le tournesol, le coton, des plantes potagères ou des plantes décoratives.

13. Utilisation selon l'une des rev. 1 à 12, dans laquelle la substance active de formule I est appliquée à une dose de 0,05 à 12 kg/ha.

14. Utilisation selon la rev. 13, dans laquelle la dose d'application est de 0,5 à 8 kg/ha.

15. Utilisation selon la rev. 13 ou 14, dans laquelle la dose d'application est de 1 à 4 kg/ha.

16. Utilisation selon l'une des rev. 10 à 12, dans laquelle la substance active de formule I est appliquée en post-levée mais avant l'apparition des épis ou des anthères.

17. Utilisation selon la rev. 16, dans laquelle l'application est faite au stade 5½ feuilles.

18. Utilisation selon la rev. 17, dans laquelle l'application est faite avant le début de la floraison.

19. Procédé pour obtenir des semences hybrides, caractérisé en ce que l'on stérilise par application d'un composé de formule I selon la rev. 1 les organes mâles d'une plante souche choisie comme plante parente femelle au début de la floraison, mais avant la formation des parties mâles des fleurs, on féconde la plante par un pollen étranger d'une plante choisie, analogue du point de vue génétique, et on récolte les semences hybrides mûres.

20. Procédé selon la rev. 19, caractérisé en ce que l'on applique la substance active à une dose de 0,05 à 12 kg/ha sur la plante parente femelle ou son habitat.